(19) Europäisches Patentamt
European Patent Office
Office européen des brevets

(11) Veröffentlichungsnummer: **0 397 044 B1**

(12) # EUROPÄISCHE PATENTSCHRIFT

(45) Veröffentlichungstag der Patentschrift: **27.12.95**

(51) Int. Cl.[6]: **C07D 213/55**, A61K 31/44, C07D 409/06, C07D 401/06, C07D 409/10, C07D 213/56

(21) Anmeldenummer: **90108433.5**

(22) Anmeldetag: **04.05.90**

(54) **Neue Arylsulfonamide, diese Verbindungen enthaltende Arzneimittel und Verfahren zu ihrer Herstellung**

(30) Priorität: **12.05.89 DE 3915506**
**28.09.89 DE 3932403**

(43) Veröffentlichungstag der Anmeldung:
**14.11.90 Patentblatt 90/46**

(45) Bekanntmachung des Hinweises auf die Patenterteilung:
**27.12.95 Patentblatt 95/52**

(84) Benannte Vertragsstaaten:
**AT BE CH DE DK ES FR GB GR IT LI LU NL SE**

(56) Entgegenhaltungen:
**EP-A- 0 098 690**
**EP-A- 0 135 316**
**EP-A- 0 287 270**
**EP-A- 0 304 271**

(73) Patentinhaber: **Dr. Karl Thomae GmbH**

**D-88397 Biberach (DE)**

(72) Erfinder: **Heckel, Armin, Dr. Dipl.-Chem.**
**Geschwister-Scholl-Strasse 71**
**D-7950 Biberach 1 (DE)**
Erfinder: **Nickl, Josef, Dr. Dipl.-Chem.**

**verstorben (DE)**
Erfinder: **Soyka, Rainer, Dr. Dipl.-Chem.**
**Schlehenhang 17/2**
**D-7950 Biberach 1 (DE)**
Erfinder: **Eisert, Wolfgang, Prof. Dr. Dr. Dr.**
**Friedrich-Goll-Weg 5**
**D-7950 Biberach 1 (DE)**
Erfinder: **Müller, Thomas, Dr. Dipl.-Chem.**
**Gymnasiumstrasse 16**
**D-7950 Biberach 1 (DE)**
Erfinder: **Weisenberger, Johannes, Dr. Dipl.-Chem.**
**Haydnweg 5**
**D-7950 Biberach 1 (DE)**
Erfinder: **Meade, Christopher, Dr.**
**Burgstrasse 104**
**D-6530 Bingen-Buedesheim (DE)**
Erfinder: **Muacevic, Gojko, Dr.**
**In der Doerrwiese 13**
**D-6507 Ingelheim am Rhein (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 397 044 B1

## Beschreibung

Gegenstand der vorliegenden Erfindung sind neue Arylsulfonamide der Formel

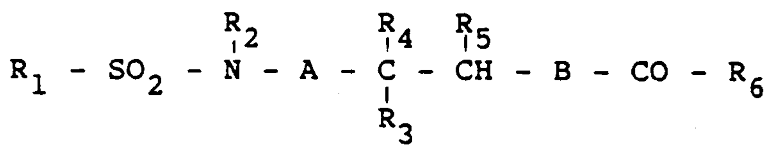 ,(I)

deren Enantiomere, deren cis- und trans-Isomere, sofern $R_4$ und $R_5$ zusammen eine Kohlenstoff-Kohlenstoff-Bindung darstellen, sowie deren Additionssalze, insbesondere für die pharmazeutische Anwendung deren physiologisch verträgliche Additionssalze mit anorganischen oder organischen Basen, falls $R_6$ eine Hydroxygruppe darstellt, welche wertvolle pharmakologische Eigenschaften aufweisen, insbesondere antithrombotische Wirkungen. Außerdem stellen die neuen Verbindungen gleichzeitig Thromboxanantagonisten (TRA) und Thromboxansynthesehemmer (TSH) dar und inhibieren somit auch die durch Thromboxan vermittelten Wirkungen. Ferner weisen diese auch eine Wirkung auf die $PGE_2$-Produktion in der Lunge und auf die $PGD_2$-, $PGE_2$- und $PGF_{2\alpha}$-Produktion in Humanthrombozyten auf.

Gegenstand der vorliegenden Erfindung sind somit die neuen Verbindungen der obigen Formel I, deren Additionssalze mit anorganischen oder organischen Basen, insbesondere für die pharmazeutische Verwendung deren physiologisch verträgliche Additionssalze, diese Verbindungen enthaltende Arzneimittel und Verfahren zu ihrer Herstellung.

In der obigen Formel bedeutet

$R_1$ eine Phenylalkyl-, Trialkylphenyl-, Tetramethylphenyl-oder Pentamethylphenylgruppe, eine gegebenenfalls durch ein Halogenatom oder eine Alkylgruppe substituierte Thienylgruppe oder eine Phenylgruppe, die durch eine Nitrogruppe monosubstituiert oder durch ein Halogenatom, eine Alkyl-, Trifluormethyl- oder Alkoxygruppe mono- oder disubstituiert sein kann, wobei die Substituenten gleich oder verschieden sein können,

$R_2$, $R_4$ und $R_5$ die gleich oder verschieden sein können, jeweils ein Wasserstoffatom oder eine Alkylgruppe oder

$R_2$ ein Wasserstoffatom oder eine Alkylgruppe und $R_4$ und $R_5$ zusammen eine Kohlenstoff-Kohlenstoff-Bindung,

$R_3$ eine gegebenenfalls durch eine Alkylgruppe substituierte Pyridylgruppe,

$R_6$ eine Hydroxy-, Alkoxy-, Amino-, Alkylamino- oder Dialkylaminogruppe,

A eine Gruppe der Formeln

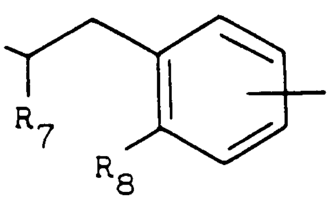 , 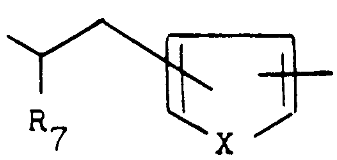 oder 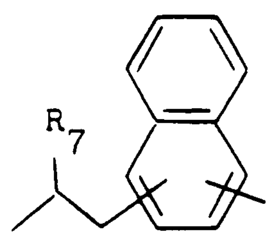 ,

in denen

$R_7$ ein Wasserstoffatom oder eine Alkylgruppe,

$R_8$ ein Wasserstoffatom oder

$R_7$ und $R_8$ zusammen eine Methylen- oder Ethylengruppe und

X eine durch eine Alkylgruppe substituierte Iminogruppe, ein Sauerstoff- oder Schwefelatom darstellen, wobei die $-CHR_7$-Gruppe mit der $-NR_2$-Gruppe verknüpft ist, und

B eine Kohlenstoff-Kohlenstoff-Bindung oder eine gegebenenfalls durch eine oder zwei Alkylgruppen substituierte geradkettige Alkylengruppe mit 1 bis 4 Kohlenstoffatomen, wobei alle vorstehend erwähnten Alkyl- und Alkoxyteile jeweils 1 bis 3 Kohlenstoffatome enthalten können.

Für die bei der Definition der Reste eingangs erwähnten Bedeutungen kommt beispielsweise für

$R_1$ die der Benzyl-, 2-Phenylethyl-, 3-Phenylpropyl-, 2,4,6-Trimethylphenyl-, 2,4,6-Triethylphenyl-, 2,4,6-Tri-n-propylphenyl-, 2,3,5,6-Tetramethylphenyl-, 3,4,5,6-Tetramethylphenyl-, 2,4,5,6-Tetramethylphenyl-, 2,3,4,5,6-Pentamethylphenyl-, 2-Thienyl-, 3-Thienyl-, 5-Methyl-2-thienyl-, 5-Ethyl-2-thienyl-, 5-n-Propyl-2-thienyl-, 5-n-Isopropyl-2-thienyl-, 5-Chlor-2-thienyl-, 5-Brom-2-thienyl-, 5-Methyl-3-thienyl-, 5-Ethyl-3-thienyl-, 5-n-Propyl-3-thienyl-, 5-n-Isopropyl-3-thienyl-, 5-Chlor-3-thienyl-, 5-Brom-3-thienyl-, Phenyl-, 2-Methylphenyl-, 3-Methylphenyl-, 4-Methylphenyl-, 2-Ethylphenyl-, 3-Ethylphenyl-, 4-Ethylphenyl-, 4-Isopropylphenyl-, 2-Trifluormethylphenyl-, 3-Trifluormethylphenyl-, 4-Trifluormethylphenyl-,2-Methoxyphenyl-, 3-Methoxyphenyl-, 4-Methoxyphenyl-, 2-Ethoxyphenyl-, 3-Ethoxyphenyl-, 4-Ethoxyphenyl-, 4-n-Propoxyphenyl-, 4-Isopropoxyphenyl-, 2-Fluorphenyl-, 3-Fluorphenyl-, 4-Fluorphenyl-, 2-Chlorphenyl-, 3-Chlorphenyl-, 4-Chlorphenyl-, 2-Bromphenyl-, 4-Bromphenyl-, 2-Nitrophenyl-, 4-Nitrophenyl-, 3,4-Dimethylphenyl-, 3,4-Dimethoxyphenyl-, 2,4-Difluorphenyl-, 2,4-Dichlorphenyl-, 2,5-Dichlorphenyl-, 2,4-Dibromphenyl-, 2,4-Ditrifluormethylphenyl-, 2-Methoxy-5-chlorphenyl- oder 2-Methyl-5-chlorphenylgruppe,

für $R_2$, $R_4$, $R_7$ und $R_8$ jeweils die des Wasserstoffatoms, der Methyl-, Ethyl-, n-Propyl- oder Isopropylgruppe,

für $R_6$ die der Hydroxy-, Methoxy-, Ethoxy-, n-Propoxy-, Isopropoxy-, Amino-, Methylamino-, Ethylamino-, Isopropylamino-, Dimethylamino-, Diethylamino-, Diisopropylamino- oder Methyl-ethylaminogruppe,

für $R_3$ die der 2-Pyridyl-, 3-Pyridyl-, 4-Pyridyl-, 4-Methyl-(2-pyridyl)-, 2-Methyl-(3-pyridyl)-, 2-Methyl-(4-pyridyl)- oder 6-Isopropyl-(2-pyridyl)-gruppe,

für X die des Sauerstoff- oder Schwefelatoms, der N-Methylimino-, N-Ethylimino- oder N-Isopropyliminogruppe und

für B die der Methylen-, Ethylen-, n-Propylen-, n-Butylen-, α-Methyl-ethylen-, α-Methyl-n-propylen-, α-Ethyl-n-propylen-, α-n-Propyl-n-propylen-, α,α-Dimethyl-n-propylen-, α,α-Diethyl-n-propylen-, ß-Methyl-n-propylen-, γ-Methyl-n-propylen-, α-Methyl-n-butylen- oder α,α-Dimethyl-n-butylengruppe, wobei die Indizies auf die Carbonylgruppe bezogen sind, in Betracht.

Bevorzugte Verbindungen der obigen Formel I sind jedoch diejenigen, in der

$R_1$ eine Benzyl-, Thienyl-, Chlorthienyl-, Dichlorphenyl-, Dimethoxyphenyl-, Tetramethylphenyl- oder Pentamethylphenylgruppe oder eine gegebenenfalls durch ein Fluor- oder Chloratom, eine Nitro-, Methyl- oder Trifluormethylgruppe substituierte Phenylgruppe,

$R_2$, $R_4$ und $R_5$ jeweils ein Wasserstoffatom oder eine Methylgruppe oder

$R_2$ ein Wasserstoffatom oder eine Methylgruppe und $R_4$ und $R_5$ zusammen eine Kohlenstoff-Kohlenstoff-Bindung,

$R_3$ eine Pyridylgruppe,

$R_6$ eine Hydroxy- oder Methoxygruppe,

A eine Gruppe der Formeln

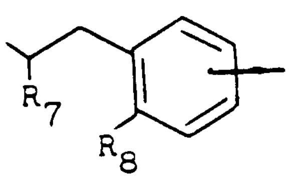 , 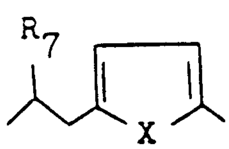 oder 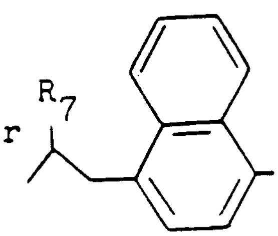 ,

in denen

$R_7$ und $R_8$ jeweils ein Wasserstoffatom oder zusammen eine Methylen- oder Ethylengruppe und

X ein Schwefelatom oder die N-Methyliminogruppe darstellen, wobei die $-CHR_7$-Gruppe mit der $-NR_2$-Gruppe verknüpft ist, und

B eine Kohlenstoff-Kohlenstoff-Bindung oder eine geradkettige Alkylengruppe mit 2 bis 4 Kohlenstoffatomen bedeuten, deren Enantiomere, deren cis- und trans-Isomeren, sofern $R_4$ und $R_5$ zusammen eine Kohlenstoff-Kohlenstoff-Bindung bilden, sowie deren Additionssalze, insbesondere für die pharmazeutische Anwendung deren physiologisch verträgliche Additionssalze mit anorganischen oder organischen Basen, falls $R_6$ eine Hydroxygruppe darstellt.

Besonders bevorzugt sind jedoch diejenigen Verbindungen der Formel I, in der

$R_1$ eine Tetramethylphenyl- oder Pentamethylphenylgruppe oder eine in 4-Stellung durch eine Methylgruppe, eine Trifluormethylgruppe, ein Fluor-, Chlor- oder Bromatom substituierte Phenylgruppe,

$R_2$, $R_4$ und $R_5$ jeweils ein Wasserstoffatom oder

$R_2$ ein Wasserstoffatom und $R_4$ und $R_5$ zusammen eine Kohlenstoff-Kohlenstoff-Bindung,

$R_3$ eine 3-Pyridylgruppe,
A eine Gruppe der Formel

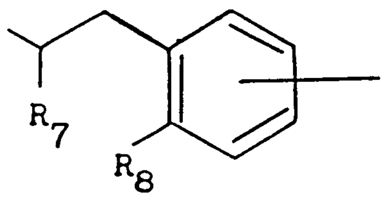

in der
$R_7$ und $R_8$ jeweils ein Wasserstoffatom oder
$R_7$ und $R_8$ zusammen eine Methylengruppe darstellen, und
$R_6$ die Hydroxygruppe bedeuten, deren Enantiomere, deren cis- und trans-Isomeren, sofern $R_4$ und $R_5$ zusammen eine Kohlenstoff-Kohlenstoff-Bindung bilden, sowie deren physiologisch verträgliche Additionssalze mit anorganischen oder organischen Basen.

Erfindungsgemäß erhält man die neuen Verbindungen nach folgenden Verfahren:

a.) Acylierung einer Verbindung der Formel

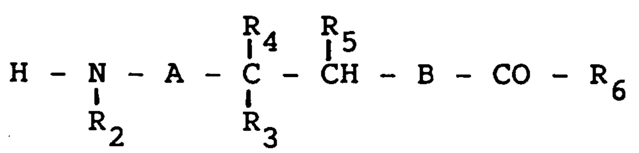
,(II)

in der
$R_2$ bis $R_6$, A und B wie eingangs definiert sind, mit einem Sulfonsäurederivat der Formel

$R_1$ - $SO_2X$ ,(III)

in der
$R_1$ wie eingangs definiert ist und
X eine nucleophile Austrittsgruppe wie ein Halogenatom oder eine Alkoxygruppe, z.B. ein Chlor- oder Bromatom, eine Methoxy- oder Äthoxygruppe, darstellt.

Die Umsetzung wird vorzugsweise in einem Lösungsmittel wie Methanol, Äthanol, Wasser/Methanol, Dioxan, Tetrahydrofuran oder Chloroform gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50 °C, vorzugsweise jedoch bei Raumtemperatur, durchgeführt.

b.) Zur Herstellung von Verbindungen der Formel I, in der $R_6$ eine Hydroxygruppe darstellt:
Abspaltung eines Schutzrestes von einer Verbindung der allgemeinen Formel

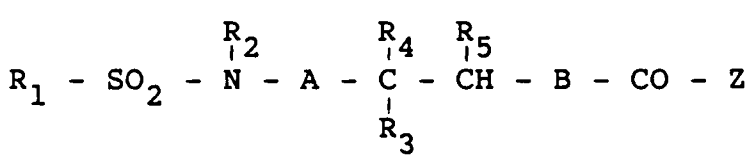
,(IV)

in der
$R_1$ bis $R_5$, A und B wie eingangs definiert sind und
Z eine hydrolytisch, thermolytisch oder hydrogenolytisch abspaltbare Schutzgruppe für eine Carboxygruppe oder ein funktionelles Derivat der Carboxygruppe darstellt.

Als hydrolysierbare Gruppen kommen beispielsweise funktionelle Derivate der Carboxygruppe wie deren unsubstituierte oder substituierte Amide, Ester, Thioester, Orthoester, Iminoäther, Amidine oder Anhydride, die Nitrilgruppe, Äthergruppen wie die Methoxy-, Ethoxy-, tert.Butoxy- oder Benzyloxygruppe

oder Lactone und
als thermolytisch abspaltbare Gruppen beispielsweise Ester mit tertiären Alkoholen, z.B. der tert.Butylester, und als hydrogenolytisch abspaltbare Gruppen beispielsweise Aralkylgruppen, z.B. die Benzylgruppe, in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/ Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Enthält beispielsweise eine Verbindung der Formel IV eine Nitril- oder Aminocarbonylgruppe, so können diese Gruppen vorzugsweise mittels 100%iger Phosphorsäure bei Temperaturen zwischen 100 und 180°C, vorzugsweise bei Temperaturen zwischen 120 und 160°C, oder auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxygruppe übergeführt werden.

Enthält beispielsweise eine Verbindung der Formel IV eine Säureamidgruppe wie die Diethylaminocarbonyl- oder Piperidinocarbonylgruppe, so kann diese Gruppe vorzugsweise hydrolytisch in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, in die Carboxygruppe übergeführt werden.

Enthält beispielsweise eine Verbindung der Formel IV die tert.Butyloxycarbonylgruppe, so kann die tert.Butylgruppe auch thermisch gegebenenfalls in einem inerten Lösungsmittel wie Methlenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 100°C, abgespalten werden.

Enthält beispielsweise eine Verbindung der Formel IV die Benzyloxy- oder Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50°C, z.B. bei Raumtemperatur und einem Wasserstoffdruck von 1 bis 5 bar abgespalten werden. Bei der Hydrogenolyse kann gleichzeitig eine halogenhaltige Verbindung enthalogeniert und eine vorhandende Doppelbindung aufhydriert werden.

c.) Zur Herstellung von Verbindungen der Formel I, in der $R_4$ und $R_5$ jeweils ein Wasserstoffatom darstellen:
Hydrierung einer Verbindung der Formel

$$R_1 - SO_2 - \overset{\displaystyle R_2}{\overset{|}{N}} - A - \underset{\displaystyle R_3}{\underset{|}{C}} = CH - B - CO - R_6 \quad ,(V)$$

in der
$R_1$ bis $R_3$, $R_6$, A und B wie eingangs definiert sind.

Die Hydrierung wird in einem geeigneten Lösungsmittel wie Methanol, Äthanol, Dioxan, Essigester oder Eisessig mit katalytisch angeregtem Wasserstoff, z.B. mit Wasserstoff in Gegenwart eines Hydrierungskatalysators wie Raney-Nickel, Palladium, Palladium/Kohle, Platin oder Platin/Kohle und einem Wasserstoffdruck von 1 bis 5 bar, oder mit nascierendem Wasserstoff, z.B. in Gegenwart von Eisen/Salzsäure, Zink/Eisessig, Zinn(II)chlorid/Salzsäure oder Eisen(II)sulfat/Schwefelsäure, bei Temperaturen zwischen 0 und 50°C, vorzugsweise bei Raumtemperatur, durchgeführt. Die katalytische Hydrierung kann jedoch auch stereoselektiv in Gegenwart eines geeigneten Katalysators erfolgen.

Hierbei kann eine gegebenenfalls im Rest $R_1$ vorhandene Nitrogruppe gleichzeitig mitreduziert oder ein gegebenenfalls im Rest $R_1$ vorhandenes Chlor- oder Bromatom durch ein Wasserstoffatom ersetzt

werden.

d.) Zur Herstellung von Verbindungen der Formel I, in der $R_4$ und $R_5$ zusammen eine Kohlenstoff-Kohlenstoff-Bindungdarstellen:

Umsetzung einer Verbindung der Formel

$$R_1 - SO_2 - \overset{R_2}{\overset{|}{N}} - A - CO - R_3 \qquad ,(VI)$$

in der

$R_1$ bis $R_3$ und A wie eingangs definiert sind, mit einer Verbindung der Formel

$$W - \overset{R_5{}'}{\overset{|}{CH}} - B - CO - R_6 \qquad ,(VII)$$

in der

B und $R_6$ wie eingangs definiert sind,

$R_5$' ein Wasserstoffatom oder eine Alkylgruppe mit 1 bis 3 Kohlenstoffatomen und

W einen Triphenylphosphoniumhalogenid-, Dialkylphosphonsäure-oder ein Magnesiumhalogenidrest bedeuten, und gegebenenfalls anschließende Dehydratisierung.

Die Umsetzung wird vorzugsweise in einem geeigneten Lösungsmittel wie Diethylether, Tetrahydrofuran, Dioxan oder Dimethylformamid bei Temperaturen zwischen -30 und 100 ° C, vorzugsweise bei Temperaturen zwischen -20 und 25 ° C, durchgeführt.

Die Umsetzung mit einem Triphenylphosphoniumhalogenid der Formel VII wird besonders vorteilhaft jedoch in Gegenwart einer Base wie Kalium-tert.butylat oder Natriumhydrid durchgeführt.

Sollte bei der Umsetzung mit einem Magnesiumhalogenid der Formel VII bei dem im Reaktionsgemisch primär entstandenen Carbinol die Hydroxygruppe nicht während der Umsetzung abgespalten werden, so wird diese in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Ethanol, Isopropanol oder Dioxan bei Temperaturen zwischen 0 und 120 ° C, z.B. bei Temperaturen zwischen Raumtemperatur und der Siedetemperatur des Reaktionsgemisches, abgespalten.

Erhält man erfindungsgemäß eine Verbindung der Formel I, in der $R_2$ ein Wasserstoffatom darstellt, so kann diese mittels Alkylierung in eine entsprechende Verbindung der Formel I, in der $R_2$ eine Alkylgruppe darstellt, übergeführt werden oder

eine Verbindung der Formel I, in der $R_6$ eine Hydroxygruppe darstellt oder enthält, so kann diese mittels Veresterung oder Amidierung in eine entsprechende Verbindung der Formel I, in der $R_6$ eine Alkoxy-, Amino-, Alkylamino- oder Dialkylaminogruppe darstellt, übergeführt werden.

Die nachträgliche Alkylierung wird vorzugsweise in einem Lösungsmittel wie Methylenchlorid, Tetrahydrofuran, Dimethylformamid oder Dimethylsulfoxid in Gegenwart eines Alkylierungsmittels wie Methyljodid, Dimethylsulfat, Ethylbromid, n-Propylbromid oder Isopropylbromid gegebenenfalls in Gegenwart eines säurebindenden Mittels wie Kaliumcarbonat bei Temperaturen zwischen 0 und 70 ° C, vorzugsweise bei Temperaturen zwischen 20 und 50 ° C, durchgeführt.

Die nachträgliche Veresterung oder Amidierung wird zweckmäßigerweise in einem Lösungsmittel, z.B. in einem Überschuß des eingesetzten Alkohols wie Methanol, Äthanol oder Isopropanol oder des eingesetzten Amins wie Ammoniak, Methylamin, n-Propylamin oder Dimethylamin, in Gegenwart eines die Säure aktivierenden Mittels wie Thionylchlorid oder Chlorwasserstoffgas bei Temperaturen zwischen 0 und 180 ° C, vorzugsweise jedoch bei der Siedetemperatur des Reaktionsgemisches, durchgeführt.

Die erhaltenen Verbindungen der Formel I können ferner in ihre Enantiomeren aufgetrennt werden. So lassen sich die erhaltenen Verbindungen der Formel I, welche nur ein optisch aktives Zentrum enthalten, nach an sich bekannten Methoden (siehe Allinger N. L. und Eliel W. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Basen, und Trennen des auf diese Weise erhaltenen

Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Basen sind z.B. die D- und L-Formen von α-Phenyl-äthylamin oder Cinchonidin.

Desweiteren lassen sich die erhaltenen Verbindungen der Formel I mit mindestens 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen. Enthält beispielsweise eine Verbindung der Formel I zwei optisch aktive Kohlenstoffatome, so erhält man die entsprechenden (R R', S S')- und (R S', S R')-Formen.

Außerdem lassen sich die so erhaltenen Verbindungen der Formel I, in denen $R_4$ und $R_5$ zusammen eine Kohlenstoff-Kohlenstoff-Bindung darstellen, mittels üblichen Methoden beispielsweise durch Chromatographie an einem Träger wie Kieselgel oder durch Kristallisation in ihre cis- und trans-Isomere überführen.

Desweiteren lassen sich die so erhaltenen neuen Verbindungen der Formel I, falls diese eine Carboxygruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der Formeln II bis VII erhält man nach literaturbekannten Verfahren bzw. sind literaturbekannt.

Eine als Ausgangsstoff verwendete Verbindung der Formel II erhält man aus einer entsprechenden N-Acylaminoverbindung durch Acylierung nach Friedel-Craft, anschließende Entacylierung und gegebenenfalls anschließende Reduktion, Hydrolyse und/oder Veresterung oder
durch Umsetzung einer entsprechenden Magnesium- oder Lithiumverbindung mit einer entsprechend substituierten Pyridinverbindung wie 3-Cyano-pyridin, Pyridin-3-aldehyd oder einem Pyridin-3-carbonsäurederivat und gegebenenfalls anschließender Oxidation.

Die als Ausgangsstoffe verwendeten Verbindungen der Formeln IV, V und VI erhält man durch Umsetzung einer entsprechenden Aminoverbindung mit einem entsprechenden Sulfonylhalogenid.

Die als Ausgangsstoffe verwendeten Verbindungen der Formel VII erhält man durch Umsetzung einer entsprechenden Halogencarbonsäure mit Triphenylphosphin oder mit einem Trialkylphosphonester.

Wie bereits eingangs erwähnt, weisen die neuen Verbindungen und deren physiologisch verträgliche Additionssalze mit anorganischen oder organischen Basen wertvolle pharmakologische Eigenschaften auf, insbesondere antithrombotische Wirkungen und eine Hemmwirkung auf die Plättchenaggregation. Außerdem stellen sie auch Thromboxanantagonisten und Thromboxansynthesehemmer dar, wobei besonders bemerkenswert ist, daß die Verbindungen der Formel I diese Wirkung gleichzeitig aufweisen. Ferner weisen diese auch eine Wirkung auf die $PGE_2$-Produktion in der Lunge und auf die $PGD_2$-, $PGE_2$- und $PGF_{2\alpha}$-Produktion in Humanthrombozyten auf.

Beispielsweise werden die neuen Verbindungen

A = 6-(2-(4-Toluolsulfonylamino)indan-5-yl)-6-(3-pyridyl)hex-5-ensäure,
B = 6-(4-(2-(4-Chlorbenzolsulfonylamino)ethyl)phenyl)-6-(3-pyridyl)hex-5-ensäure,
C = 6-(2-(4-Chlorbenzolsulfonamino)indan-5-yl)-6-(3-pyridyl)hexansäure,
D = 6-(4-(2-(4-Chlorbenzolsulfonylamino)ethyl)phenyl)-6-(3-pyridyl)hexansäure und
E = 6-(4-(2-(4-Trifluormethylbenzolsulfonylamino)ethyl)phenyl)-6-(3-pyridyl)hex-5-ensäure

auf ihre biologischen Eigenschaften wie folgt geprüft:

1. Antithrombotische Wirkung

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.
Vor der Collagen-Zugabe wird das Plasma jeweils 10 Minuten mit der Substanz bei 37 ° C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz | $EC_{50}$ [µMol/l] |
|---|---|
| A | 0,3 |
| B | 0,15 |
| C | 0,3 |
| D | 1,2 |
| E | 0,1 |

2. Thromboxanantagonistische Wirkung

Venöses Humanblut wird mit 13 mM Na3-Citrat antikoaguliert und 10 Minuten bei 170 x g zentrifugiert. Das überstehende plättchenreiche Plasma wird zur Entfernung der Plasmaproteine über eine Sepharose-2B-Säule gegeben. Aliquote der erhaltenen Plättchensuspension werden mit der Testsubstanz, dem Liganden (3H-markiert) und einem Marker (14C-markiert) 60 Minuten bei Raumtemperatur inkubiert und danach 20 Sekunden bei 10 000 x g abzentrifugiert. Der Überstand wird abgezogen und das Pellet in NaOH gelöst. Die 3H-Aktivität im Überstand entspricht dem freien Liganden, 14C ergibt die Konzentration des Markers. 3H im Pellet entspricht dem gebundenen Liganden, 14C wird zur Korrektur für Ligand im Extrazellulärraum verwendet. Aus den Bindungswerten für verschiedene Konzentrationen der Testsubstanz wird nach Iteration die Verdrängungskurve ermittelt und die $IC_{50}$ bestimmt.

| Substanz | $IC_{50}$ [µMol/l] |
|---|---|
| A | 0,023 |
| B | 0,02 |
| C | 0,08 |
| D | 0,08 |
| E | 0,028 |

3. Bestimmung der Hemmwirkung auf die Thromboxansynthetase

Venöses Humanblut wird mit 13 mM Na3-Citrat antikoaguliert und 10 Minuten bei 170 x g zentrifugiert. Das überstehende plättchenreiche Plasma wird zur Entfernung der Plasmaproteine über eine Sepharose-2B-Säule gegeben. Aliquots der erhaltenen Plättchensuspension werden mit der Testsubstanz bzw. Lösungsmittel als Kontrolle 10 Minuten bei Raumtemperatur inkubiert und nach Zugabe 14C-markierter Arachidonsäure die Inkubation weitere 10 Minuten fortgesetzt. Nach Abstoppen mit 50 µl Zitronensäure wird 3 x mit 500 µl Essigester extrahiert und die vereinigten Extrakte mit Stickstoff abgeblasen. Der Rückstand wird in Essigester aufgenommen, auf DC-Folie aufgetragen und mit Chloroform:Methanol:Eisessig:Wasser (90:8:1:0,8, v/v/v/v) getrennt. Die getrockneten DC-Folien werden 3 Tage auf Röntgenfilm gelegt, die Autoradiogramme entwickelt und mit ihrer Hilfe die aktiven Zonen auf der Folie markiert. Nach Ausschneiden wird die Aktivität im Szintillationszähler bestimmt und die Hemmung der TXB2-Bildung berechnet. Die $IC_{50}$ wird durch lineare Interpolation bestimmt.

| Substanz | $IC_{50}$ [µMol/l] |
|---|---|
| A | 0,003 |
| B | 0,0008 |
| C | 0,003 |
| D | 0,001 |
| E | 0,006 |

4. Hemmung des durch U-46619 erzeugten Bronchospasmus

Mit Ethylurethan narkotisierte Meerschweinchen, welche unter Druckbegrenzung künstlich beatmet werden, erhielten wiederholt intravenöse Injektionen von dem Thromboxan-Mimetikum U-46619 (= [1R-[1α,4α,5ß(Z),6α(1E,3S*)]]-7-[6-(3-Hydroxy-1-octenyl)-2-oxabicyclo[2.2.1]hept-5-yl]-5-heptensäure). Die hierdurch hervorgerufenen Bronchospasmen werden nach einer Modifikation der Methode von Konzett und Rössler (Konzett, H. und Rössler R., Arch. exp. Pathol. u. Pharmakol. 195, 71-74 (1940)) plethysmographisch registriert. Die ausgewählte Dosis von U-46619 (2,5 - 25 µg/kg i.v.) vermindert das Atemzugvolumen um 60 % oder mehr. 10 Minuten vor dem Thromboxan-Mimetikum werden wiederholt ansteigende Dosen der zu untersuchenden Substanzen intravenös injiziert. Die prozentuale Hemmung der Verminderung des Atemzugvolumens wird durch einen Vergleich der U-46619 Wirkung vor und nach verschiedenen Dosen der zu untersuchenden Substanzen gemessen. Die nachfolgende Tabelle enthält die gefundenen $ED_{50}$-Werte, welche graphisch ermittelt wurden:

| Substanz | $ED_{50}$ [µg/kg] |
|---|---|
| A | 30 |
| B | 29 |

5. Hemmung des lethalen Effektes von Endotoxin

Männliche Sprague-Dawley Ratten werden zuerst durch intravenöse Injektion mit 0,1 mg/kg Endotoxin (Lipopolysacharid aus E.coli 0111:B4) eine Woche vor dem Hauptversuch vorbehandelt. Im Hauptversuch wird eine potentiell lethale Dosis von E.coli (40 mg/kg) intravenös injiziert und die darauffolgende Mortalität wurde über eine Beobachtungszeit von sieben Tagen registriert.

Die geprüften Tiere erhalten die zu untersuchende Substanz B eine Stunde vor und 4, 8, 24 und 48 nach der zweiten Injektion von Endotoxin als Suspension in 0,5%iger Tylose per os appliziert. Die nachfolgende Tabelle enthält die gefundenen Werte:

| Applikationsdosis der Substanz B appliziert bei jedem Dosierzeitpunkt (mg/kg) | lebend/gesamt Ratten | |
|---|---|---|
| | nach 2 Tagen | nach 7 Tagen |
| 0 | 2/10 | 2/10 |
| 1 | 7/10 | 6/10 |
| 10 | 8/10 | 5/10 |

6. Hemmung des durch die Arachidonsäure erzeugten Bronchospasmus

Mit Ethylurethan narkotisierte Meerschweinchen, welche unter Druckbegrenzung künstlich beatmet werden, erhalten eine intravenöse Injektion mit Arachidonsäure (Vorprodukt für Thromboxan) und die auftretenden Bronchospasmen werden nach einer Modifikation der Methode von Konzett und Rössler registriert. Die Dosen für die Arachidonsäure (0,5 - 2,0 mg/kg) werden so gewählt, daß eine Reduktion des Atemzugvolumens um 60 % auftritt. Aufsteigende Dosen der zu untersuchenden Substanz B werden 10 Minuten vor der Arachidonsäure injiziert. Die prozentuale Hemmung der Einschränkung des Atemzugvolumens wird durch einen Vergleich zwischen der maximalen Verminderung nach Applikation der Arachidonsäure und dem entsprechenden Wert nach Vorbehandlung mit der zu untersuchenden Substanz ermittelt. Der $ED_{50}$-Wert für die Substanz B, welcher graphisch ermittelt wurde, beträgt 8,1 µg/kg.

7. Hemmung der antigeninduzierten Anaphylaxie

Männliche Meerschweinchen werden mit 40 mg/kg i.p. Ovalbumin, adsorbiert an Aluminiumhydroxyd als Adjuvans, sensibilisiert. Ungefähr 6 Wochen später wird den gleichen Tieren eine subkutane Injektion von 0,1 mg/kg Mepyraminhydrochlorid verabreicht, um die Histaminkomponente der anaphylaktischen Reaktion, welche ansonsten bei Meerschweinchen sehr ausgeprägt ist, zu vermindern. 30 Minuten später werden die Tiere für 90 Sekunden einer vernebelten 3%igen Ovalbuminlösung in 0,9%iger Kochsalzlösung, ausgesetzt. Zehn Minuten nach Inhalationsbeginn, werden die Tiere mit einem Nackenschlag getötet und die Lungen schnell herauspräpariert. Ihr Volumen, das sogenannte "Relaxationsvolumen" wird gemessen. Die Folge der Anaphylaxie bzw. der Bronchokonstriktion ist mit einer Vergrößerung des "Relaxationsvolumens" (siehe Drazen, I.M. und Austen, K.F. in J. Appl. Physiol. 39, 916-919 (1975)) verbunden.

Die nachfolgende Tabelle enthält die gefundenen Werte:

| Tiere | Mittlere Relaxationsvolumen der Lunge (ml) | |
|---|---|---|
| | Erste Prüfung | Zweite Prüfung |
| Meerschweinchen nach Inhalation, ohne Prüfsubstanz | 7,52 (n = 6) | 7,51 (n = 6) |
| Meerschweinchen 60 Minuten vor der Inhalation vorbehandelt mit 2,5 mg/kg p.o. Substanz B | 4,34 (n = 6) | 3,81 (n = 6) |

Nichtsensibilisierte Tiere, die dem Ovalbumin ausgesetzt waren, oder sensibilisierte Meerschweinchen, welche mit einem Kontrollaerosol (Kochsalzlösung) behandelt worden waren, zeigen in allen Fällen ein Relationsvolumen von 1,5 ml oder weniger.

8. Beeinflussung der Bildung von Thromboxan und $PGE_2$ in der isolierten Lunge

Meerschweinchen werden durch einen Nackenschlag getötet, die Lunge wird schnell herauspräpariert und über die Arteria pulmonalis mit Tyrode-Lösung gewaschen. Mit der gleichen Lösung wird die Lunge perfundiert (0,5 ml/Min.) und mit einem negativen Druck (Frequenz: 52 Atemzüge/Min., Maximaldruck: -20 cm $H_2O$) beatmet. Zwei 0,1 ml Bolusinjektionen von Bradykinin (0,2 µM) werden mit ca. 60-minütigem Zeitabstand über die A. pulmonalis injiziert. 30 Minuten vor der 2. Bradykininapplikation wurde der Perfusionslösung zur kontinuierlichen Perfusion 1 µM der zu untersuchenden Substanz B zugesetzt. Die Kontroll-Lungen werden ohne Substanz perfundiert. Das Perfusat wird 2 Minuten vor den Bradykinin-Applikationen und 10 Minuten danach gesammelt. Die Proben bleiben 20 Minuten bei Raumtemperatur stehen (Umwandlung von Thromboxan $A_2$ zu $B_2$) und werden dann bei -20 °C eingefroren. Die Konzentrationen von Thromboxan $B_2$ und $PGE_2$ werden mit Hilfe eines Radioimmunoassays bestimmt. Die nachfolgenden Ergebnisse belegen, daß 1 µM Substanz B im Lungenperfusat eine Thromboxanbildung verhindert, während die $PGE_2$-Bildung gefördert wird:

| Substanz anwesend im Perfusat bei 2. Bradykinin-Applikation | Verhältnis Mediatorfreisetzung | nach 2. Bradykinin-Applikation / nach 1. Bradykinin-Applikation |
|---|---|---|
| | Thromboxan $B_2$ | $PGE_2$ |
| 1 µM B<br>Kontrolle | 0,0 0,0<br>1,18 0,50<br>0,84 | 5,08 2,23<br>1,28 1,12 1,09 |

9. Akute Toxizität

Die akute Toxizität der zu untersuchenden Substanzen wurde orientierend an Gruppen von je 10 mäusen nach oraler Gabe einer Einzeldosis bestimmt (Beobachtungszeit: 14 Tage):

| Substanz | Orientierende akute Toxizität |
|---|---|
| A | 250 mg/kg (0 von 10 Tieren gestorben) |
| B | 250 mg/kg (0 von 10 Tieren gestorben) |
| C | 250 mg/kg (0 von 10 Tieren gestorben) |
| D | 250 mg/kg (0 von 10 Tieren gestorben) |
| E | 250 mg/kg (0 von 10 Tieren gestorben) |

Aufgrund ihrer pharmakologischen Eigenschaften eignen sich die neuen Verbindungen und deren physiologisch verträgliche Additionssalze zur Behandlung und zur Prophylaxe thrombo-embolischer Erkrankungen wie Coronarinfarkt, Cerebralinfarkt, sogen. transient ischaemic attacks, Amaurosis fugax, zur Prophylaxe der Arteriosklerose und zur Metastasenprophylaxe sowie zur Behandlung der Ischämie, des Asthmas und von Allergien.

Die neuen Verbindungen und deren physiologisch verträglichen Additionssalze sind auch bei der Behandlung von Erkrankungen nützlich, bei denen eine thromboxanvermittelte Konstriktion oder $PGE_2$ vermittelte Dilatation der Kapillaren eine Rolle spielen, z.B. bei der pulmonalen Hypertension. Außerdem können diese zur Verminderung des Schweregrades einer Transplantatabstoßung, zur Verminderung der renalen Toxizität von Substanzen wie Cyclosporin, zur Behandlung von Nierenerkrankungen, insbesondere zur Therapie oder Prophylaxe von Nierenveränderungen im Zusammenhang von Hypertension, systemischem Lupus oder Ureterobstruktionen und bei Schockzuständen im Zusammenhang mit Sepsis, Trauma oder Verbrennungen eingesetzt werden.

Die zur Erzielung einer entsprechenden Wirkung erforderliche Dosierung beträgt zweckmäßigerweise zwei- bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zübereitungen wie Tabletten, Dragées, Kapseln, Pulver, Suspensionen oder Zäpfchen einarbeiten.

Ein weiterer Gegenstand der vorliegenden Erfindung sind neue Arzneimittel, enthaltend eine erfindungsgemäß hergestellte Verbindung der Formel I und einen PDE-Hemmer oder ein Lysemittel.

Als PDE-Hemmer kommt hierbei beispielsweise
2,6-Bis(diäthanolamino)-4,8-dipiperidino-pyrimido[5,4-d]pyrimidin (Dipyridamol),
2,6-Bis(diäthanolamino)-4-piperidino-pyrimido[5,4-d]pyrimidin (Mopidamol),
2-(4-Methoxy-phenyl)-5(6)-(5-methyl-3-oxo-4,5-dihydro-2H-6-pyridazinyl)-benzimidazol (Pimobendan),
2-(4-Hydroxy-phenyl)-5(6)-(5-methyl-3-oxo-4,5-dihydro-2H-6-pyridazinyl)-benzimidazol,
1-(1-Oxido-thiomorpholino)-3-piperazino-5-methyl-isochinolin,
6-[4-(3,4-Dichlorphenylsulfinyl)-butoxy]-3,4-dihydrocarbostyril und
6-[4-(2-Pyridylsulfonyl)-butoxy]carbostyril, wobei die perorale Tagesdosis
für Dipyridamol 2,5 bis 7,5 mg/kg, vorzugsweise 5 mg/kg,
für Mopidamol 15 bis 25 mg/kg, vorzugsweise 20 mg/kg,
für 2-(4-Methoxy-phenyl)-5(6)-(5-methyl-3-oxo-4,5-dihydro-2H-6-pyridazinyl)-benzimidazol 0,05 bis 0,15 mg/kg, vorzugsweise 0,08 bis 0,10 mg/kg,
für 2-(4-Hydroxy-phenyl)-5(6)-(5-methyl-3-oxo-4,5-dihydro-2H-6-pyridazinyl)-benzimidazol 0,05 bis 0,15 mg/kg, vorzugsweise 0,08 bis 0,10 mg/kg,
für 1-(1-Oxido-thiomorpholino)-3-piperazino-5-methyl-isochinolin 0,20 bis 2,00 mg/kg, vorzugsweise 0,40 bis 1,00 mg/kg,
für 6-[4-(3,4-Dichlorphenylsulfinyl)-butoxy]-3,4-dihydrocarbostyril 0,10 bis 1,00 mg/kg, vorzugsweise 0,20 bis 0,50 mg/kg und
für 6-[4-(2-Pyridylsulfonyl)-butoxy]carbostyril 0,10 bis 1,00 mg/kg, vorzugsweise 0,20 bis 0,50 mg/kg, beträgt,
und als Lysemittel Plasminogen-Aktivatoren wie t-PA, rt-PA, Streptokinase, Eminase oder Urokinase in Betracht, wobei die Lysemittel parenteral, vorzugsweise jedoch intravenös, verabreicht werden, z.B. t-PA oder rt-PA in einer Dosierung zwischen 15 und 100 mg pro Patient, Urokinase in einer Dosis zwischen 250 000 und 3 000 000 Einheiten pro Patient, Eminase in einer Dosierung von ungefähr 30 mg pro Patient und Streptokinase in einer Dosis zwischen 5 x $10^4$ bis 3 x $10^7$ IU jeweils innerhalb 5 Minuten und 24 Stunden.

Zur pharmazeutischen Anwendung läßt sich eine neue Kombination, enthaltend 1 bis 500 mg eines PDE-Hemmers, vorzugsweise jedoch 2 bis 75 mg, plus 10 bis 300 mg einer erfindungsgemäß hergestellten Verbindung der Formel I, vorzugsweise jedoch 10 bis 200 mg, sowie deren physiologisch verträgliche Additionssalze zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten. Hierbei erfolgt die Applikation am Erwachsenen zur Erzielung einer entsprechenden Wirkung 2 bis 4 mal täglich, vorzugsweise jedoch 3 bis 4 mal täglich.

Desweiteren läßt sich zur pharmazeutischen Anwendung eine neue Kombination, enthaltend ein Lysemittel in den vorstehend erwähnten Dosierungen plus 10 bis 300 mg einer erfindungsgemäß hergestellten Verbindung der Formel I, vorzugsweise jedoch 10 bis 200 mg, sowie deren physiologisch verträgliche Additionssalze in die üblichen parenteralen, vorzugsweise in die üblichen intravenösen, Applikationsformen wie Ampullen oder Infusionen einarbeiten, wobei die Applikation innerhalb von 5 Minuten und 24 Stunden erfolgen kann.

Selbstverständlich können die einzelnen Wirksubstanzen der vorstehenden Kombinationen gewünschtenfalls appliziert werden.

Die nachfolgenden Beispiele sollen die Erfindung näher erläutern:

## Beispiel 1

### 6-(4-(2-(4-Chlorbenzolsulfonylamino)ethyl)phenyl)-6-(3-pyridyl)hex-5-ensäure

a) 2-(p-Chlorbenzolsulfonylamino)ethylbenzol

Zu einer Mischung aus 150 ml Ethylenchlorid und 150 ml Wasser gibt man 30,3 g 2-Phenylethylamin, 12 g Natriumhydroxid und 0,5 g Tetrabutylammoniumbromid. Unter Rühren versetzt man die Mischung portionsweise mit 65,5 g 4-Chlorbenzolsulfonsäurechlorid. Nach 30 Minuten wird die organische Phase abgetrennt, eingeengt und der Rückstand aus Toluol umkristallisiert.

Ausbeute: 65 g (88 % der Theorie),
Schmelzpunkt: 90 ° C

b) 4-(2-(4-Chlorbenzolsulfonylamino)ethyl)phenyl-3-pyridylketon

100 g Aluminiumtrichlorid werden langsam mit 25,5 ml Dimethylformamid versetzt, so daß die Temperatur 70 ° C nicht überschreitet. Zu dieser Mischung gibt man 35,6 g Nicotinsäurechlorid-hydrochlorid und 49 g 2-(4-Chlorbenzolsulfonylamino)ethylbenzol und erhitzt 2 Stunden auf 100 ° C. Die Reaktionsmischung wird auf Eis gegeben, neutralisiert und mit Ethylenchlorid extrahiert. Die organische Phase wird eingeengt und der Rückstand über eine Kieselgelsäule mit Ethylenchlorid/Ethanol (40:1) chromatographiert.
Ausbeute: 16,7 g (25 % der Theorie),
Schmelzpunkt: 150-152 ° C

| $C_{20}H_{17}ClN_2O_3S$ (400,91) | | | | | | |
|---|---|---|---|---|---|---|
| Ber.:<br>Gef.: | C | 59,92<br>60,06 | H | 4,28<br>3,98 | N | 6,99<br>6,87 |

c) 6-(4-(2-(4-Chlorbenzolsulfonylamino)ethyl)phenyl)-6-(3-pyridyl)hex-5-ensäure

Zu einer Suspension aus 6,7 g 4-Carboxybutyl-triphenylphosphoniumbromid und 4,5 g Kalium-tert.butylat in 100 ml Tetrahydrofuran gibt man bei 0 ° C 4,0 g 4-(2-(4-Chlorbenzolsulfonylamino)ethyl)-phenyl-3-pyridylketon und rührt 2 Stunden. Die Reaktionsmischung wird mit Eiswasser zersetzt und mit Toluol gewaschen. Die wässerige Phase wird angesäuert und mit Ethylenchlorid extrahiert. Der organische Extrakt wird eingeengt und der Rückstand über eine Kieselgelsäule mit Ethylenchlorid/Ethanol (20:1) chromatographiert. Die produkthaltige Fraktion wird eingeengt, der Rückstand in Essigester gelöst und durch Zugabe von 2 ml Cyclohexylamin das Cyclohexylammonium-Salz gefällt.
Ausbeute: 1,9 g (36 % der Theorie),
Schmelzpunkt: 95 ° C (Zers.)

| $C_{25}H_{25}ClN_2O_4S$ x 1/2 Cyclohexylamin (534,61) | | | | | | |
|---|---|---|---|---|---|---|
| Ber.:<br>Gef.: | C | 62,91<br>62,80 | H | 5,94<br>6,03 | N | 6,55<br>6,72 |

Beispiel 2

6-(1-(4-(2-(4-Chlorbenzolsulfonylamino)ethyl)naphthyl))-6-(3-pyridyl)hex-5-ensäure

a) 1-(2-(p-Chlorbenzolsulfonylamino)ethyl)naphthalin

Hergestellt aus 1-(2-Aminoethyl)naphthalin und 4-Chlorbenzolsulfonsäurechlorid analog Beispiel 1a. Die Reinigung des Rohproduktes erfolgte durch Säulenchromatographie an Kieselgel mit Ethylenchlorid/Cyclohexan (2:1).
Ausbeute: 92 % der Theorie,
Schmelzpunkt: 98-99 ° C

| $C_{18}H_{16}ClNO_2S$ (345,87) | | | | | | |
|---|---|---|---|---|---|---|
| Ber.:<br>Gef.: | C | 62,51<br>62,39 | H | 4,66<br>4,68 | N | 4,05<br>3,86 |

b) 4-(2-(4-Chlorbenzolsulfonylamino)ethyl)naphthyl-3-pyridylketon

Hergestellt aus Nicotinsäurechlorid-hydrochlorid und 1-(2-(p-Chlorbenzolsulfonylamino)ethyl)naphthalin analog Beispiel 1b. Die Reinigung des Rohproduktes erfolgte durch Säulenchromatographie an Kieselgel mit Ethylenchlorid/Essigester (5:1).
Ausbeute: 22 % der Theorie,
Harz, $R_f$-Wert: 0,41 (Kieselgel: Ethylenchlorid/Essigsäureethylester = 3:1)

| $C_{24}H_{19}ClN_2O_3S$ (450,96) | | | | | | |
|---|---|---|---|---|---|---|
| Ber.:<br>Gef.: | C | 63,92<br>63,54 | H | 4,25<br>4,43 | N | 6,21<br>6,01 |

c) 6-(1-(4-(2-(4-Chlorbenzolsulfonylamino)ethyl)naphthyl))-6-(3-pyridyl)hex-5-ensäure

Hergestellt aus 4-(2-(4-Chlorbenzolsulfonylamino)ethyl-naphthyl-3-pyridylketon und 4-Carboxybutyl-triphenylphosphoniumbromid analog Beispiel 1c, jedoch ohne Salzfällung mit Cyclohexylamin.
Ausbeute: 43 % der Theorie,
Harz, $R_f$-Wert: 0,52 (Kieselgel: Ethylenchlorid/Essigsäureethylester = 4:1)

| $C_{29}H_{27}ClN_2O_4S$ (535,09) | | | | | | |
|---|---|---|---|---|---|---|
| Ber.:<br>Gef.: | C | 65,10<br>64,91 | H | 5,09<br>5,35 | N | 5,24<br>5,20 |

Beispiel 3

6-(5-(2-(4-Fluorbenzolsulfonylamino)ethyl)-N-methyl-pyrrol-2-yl)-6-(3-pyridyl)hex-5-ensäure

a) 5-(2-(4-Fluorbenzolsulfonylamino)ethyl)-N-methyl-pyrrol-2-yl-3-pyridylketon

Eine Lösung von 14,1 g 2-(2-(4-Fluorbenzolsulfonylamino)ethyl)-N-methyl-pyrrol in 100 ml Toluol und 50 ml Dimethylformamid wird portionsweise mit 9,8 g Nicotinsäurechloridhydrochlorid versetzt. Man kocht 2 Stunden unter Rückfluß, gibt die Reaktionsmischung auf Eis, neutralisiert und extrahiert mit Ethylenchlorid. Das Rohprodukt wird über eine Kieselgelsäule mit Ethylenchlorid/Ethanol (20:1) chromatographiert.
Ausbeute: 4,6 g (24 % der Theorie),
Schmelzpunkt: 140 °C

| $C_{19}H_{18}FN_3O_3S$ (387,44) | | | | | | |
|---|---|---|---|---|---|---|
| Ber.:<br>Gef.: | C | 58,90<br>58,62 | H | 4,68<br>4,52 | N | 10,85<br>10,70 |

b) 6-(5-(2-(4-Fluorbenzolsulfonylamino)ethyl)-N-methyl-pyrrol-2-yl)-6-(3-pyridyl)hex-5-ensäure

Hergestellt aus 5-(2-(4-Fluorbenzolsulfonylamino)ethyl)N-methyl-pyrrol-2-yl-3-pyridylketon und 4-Carboxybutyltriphenylphosphoniumbromid analog Beispiel 1c, das Rohprodukt wird jedoch durch Umkristallisation aus Wasser/Isopropanol gereinigt.
Ausbeute: 55 % der Theorie,
Schmelzpunkt: 190 °C

| $C_{24}H_{26}FN_3O_4S$ (471,56) | | | | | | |
|---|---|---|---|---|---|---|
| Ber.: Gef.: | C | 61,13 61,23 | H | 5,56 5,72 | N | 8,91 9,00 |

Beispiel 4

6-(5-(2-(4-Chlorbenzolsulfonylamino)ethyl)thiophen-2-yl)-6-(3-pyridyl)hex-5-ensäure

a) 2-(2-(p-Chlorbenzolsulfonylamino)ethyl)thiophen

Hergestellt aus 2-(2-Aminoethyl)-thiophen und 4-Chlorbenzolsulfon-säurechlorid analog Beispiel 1a.
Ausbeute: 69 % der Theorie,
Schmelzpunkt: 93 ° C

| $C_{12}H_{12}ClNO_2S_2$ (301,83) | | | | | | |
|---|---|---|---|---|---|---|
| Ber.: Gef.: | C | 47,75 47,75 | H | 4,01 3,88 | N | 4,64 4,45 |

b) 5-(2-(4-Chlorbenzolsulfonylamino)ethyl)thiophen-2-yl-3-pyridylketon

Zu einer Suspension aus 20 g Aluminiumtrichlorid und Nicotinsäurechlorid-hydrochlorid in 150 ml Ethylenchlorid tropft man eine Lösung von 15 g 2-(2-(4-Chlorbenzolsulfonylamino)ethyl)thiophen in 50 ml Ethylenchlorid zu. Man erwärmt 1 1/2 Stunden auf 50 ° C, gibt die Reaktionsmischung auf Eis, saugt den Niederschlag ab und kristallisiert aus Methanol um.
Ausbeute: 3,7 g (17 % der Theorie),
Schmelzpunkt: 154-160 ° C

| $C_{18}H_{15}ClN_2O_3S$ x 1/2 HCl (433,06) | | | | | | |
|---|---|---|---|---|---|---|
| Ber.: Gef.: | C | 49,92 50,29 | H | 3,58 3,82 | N | 6,47 6,38 |

c) 6-(5-(2-(4-Chlorbenzolsulfonylamino)ethyl)thiophen-2-yl)-6-(3-pyridyl)hex-5-ensäure

Hergestellt aus 5-(2-(4-Chlorbenzolsulfonylamino)ethyl)thiophen-2-yl-3-pyridylketon und 4-Carboxybutyl-triphenylphosphoniumbromid analog Beispiel 1c, nach der Säulenchromatographie wird jedoch aus Essigester umkristallisiert.
Ausbeute: 20 % der Theorie,
Schmelzpunkt: 138 ° C

| $C_{23}H_{23}ClN_2O_4S$ (491,04) | | | | | | |
|---|---|---|---|---|---|---|
| Ber.: Gef.: | C | 56,26 56,24 | H | 4,72 4,67 | N | 5,71 5,70 |

Beispiel 5

6-(2-(4-Chlorbenzolsulfonylamino)tetralin-6- und 7-yl)-6-(3-pyridyl)hex-5-ensäure

a) 2-Acetylaminotetralin-6- und 7-yl-3-pyridylketon

Hergestellt aus 2-Acetylaminotetralin und Nicotinsäurechlorid-hydrochlorid analog Beispiel 1b.
Ausbeute: 35 % der Theorie,
Harz, $R_f$-Wert: 0,28 (Kieselgel: Ethylenchlorid/Ethanol = 10:1)

| $C_{18}H_{18}N_2O_2$ (294,40) | | | | | | |
|---|---|---|---|---|---|---|
| Ber.:<br>Gef.: | C | 73,45<br>73,38 | H | 6,16<br>6,23 | N | 9,52<br>9,26 |

b) 2-(4-Chlorbenzolsulfonylamino)tetralin-6- und 7-yl-3-pyridylketon

Das Gemisch aus 2-Acetylaminotetralin-6- und 7-yl-3-pyridylketon wird 20 Stunden in 150 ml konzentrierter Salzsäure unter Rückfluß gekocht. Das Lösungsmittel wird abgezogen und der Rückstand mit 4-Chlorbenzolsulfonsäurechlorid gemäß Beispiel 1a behandelt.
Ausbeute: 35 % der Theorie,
Schmelzpunkt: 152-155 °C (Essigester)

| $C_{22}H_{19}ClO_3S$ (426,94) | | | | | | |
|---|---|---|---|---|---|---|
| Ber.:<br>Gef.: | C | 61,89<br>61,92 | H | 4,49<br>4,45 | N | 6,56<br>6,46 |

c) 6-(2-(4-Chlorbenzolsulfonylamino)tetralin-6- und 7-yl)-6-(3-pyridyl)hex-5-ensäure

Hergestellt aus dem Gemisch von 2-(4-Chlorbenzolsulfonylamino)tetralin-6- und 7-yl-3-pyridylketon und 4-Carboxybutyltriphenyl-phosphoniumbromid analog Beispiel 1c, jedoch ohne Salzfällung mit Cyclohexylamin.
Ausbeute: 93 % der Theorie,
Harz, $R_f$-Wert: 0,30 (Kieselgel: Ethylenchlorid/Ethanol = 10:1)

| $C_{27}H_{27}ClN_2O_4S$ (511,07) | | | | | | |
|---|---|---|---|---|---|---|
| Ber.:<br>Gef.: | C | 63,46<br>63,29 | H | 5,33<br>5,31 | N | 5,48<br>5,22 |

Beispiel 6

6-(5-(2-(4-Fluorbenzolsulfonylamino)ethyl)-N-methyl-pyrrol-2-yl)-6-(3-pyridyl)hexansäure

Eine Mischung aus 2,36 g 6-(5-(2-(4-Fluorbenzolsulfonylamino)ethyl)-N-methyl-pyrrol-2-yl)-6-(3-pyridyl)-hex-5-ensäure, 0,4 g Natriumhydroxid und 1 g 10%iger Palladium/Kohle in 50 ml Methanol wird bei 5 bar Wasserstoffdruck hydriert. Anschließend wird vom Katalysator abfiltriert, eingeengt, der Rückstand mit Wasser verdünnt, angesäuert und mit Ethylenchlorid extrahiert. Der organische Extrakt wird eingeengt und der Rückstand aus Essigester umkristallisiert.
Ausbeute: 2 g (85 % der Theorie),
Schmelzpunkt: 146-149 °C.

| | | | | | | |
|---|---|---|---|---|---|---|
| Ber.:<br>Gef.: | C | 60,88<br>61,11 | H | 5,96<br>6,02 | N | 8,87<br>8,93 |

Beispiel 7

6-(2-(4-Toluolsulfonylamino)indan-5-yl)-6-(3-pyridyl)hex-5-ensäure

a) 2-Acetylaminoindan-5-yl-3-pyridylketon

Zu 150 g Aluminiumchlorid und 31 ml Dimethylformamid gibt man bei 70 °C portionsweise 42,7 g Nicotinsäurechlorid-hydrochlorid. Zu dieser Mischung werden portionsweise 35 g 2-Acetylaminoindan gegeben. Nach weiterem Erwärmen der Mischung bei 80 °C wird nach 2 Stunden abgekühlt und die Mischung auf 200 g Eis und 100 ml konzentrierte Salzsäure gegeben. Die saure Lösung wird vorsichtig mit Natronlauge neutralisiert und dann mit 4 x 250 ml Chloroform extrahiert. Die organischen Phasen werden gesammelt, über Natriumsulfat getrocknet und einrotiert.
Ausbeute: 43 g (76 % der Theorie),
Schmelzpunkt: 165-167 °C

| $C_{17}H_{16}N_2O_2$ (280,32) | | | | | | |
|---|---|---|---|---|---|---|
| Ber.:<br>Gef.: | C | 72,84<br>72,70 | H | 5,75<br>5,72 | N | 9,99<br>9,75 |

b) 2-Aminoindan-5-yl-3-pyridylketon

51 g 2-Acetylamino-indan-5-yl-3-pyridylketon werden mit 250 ml halbkonzentrierter Salzsäure 16 Stunden am Rückfluß erhitzt. Die Lösung wird konzentriert und dann mit 15 N Natronlauge auf pH 12 eingestellt. Der ausfallende Niederschlag wird mit Wasser gewaschen und aus 100 ml Isopropanol umkristallisiert.
Ausbeute: 42 g (97 % der Theorie),
Schmelzpunkt: 205 °C (Zers.)

| $C_{15}H_{14}N_2O$ (238,29) | | | | | | |
|---|---|---|---|---|---|---|
| Ber.:<br>Gef.: | C | 75,61<br>75,44 | H | 5,92<br>6,04 | N | 11,75<br>11,85 |

c) 2-(4-Toluolsulfonylamino)indan-5-yl-3-pyridylketon

21 g 2-Aminoindan-5-yl-3-pyridylketon werden zusammen mit 18,9 g p-Toluolsulfonsäurechlorid in 250 ml Methylenchlorid gelöst. Anschließend tropft man 9,2 g Triethylamin zu. Nach 4 Stunden wird die Suspension zur Trockene einrotiert. Der Rückstand wird in Wasser suspendiert, mit Natronlauge alkalisch gestellt und dann abgesaugt.
Ausbeute: 30,4 g (88 % der Theorie),
Schmelzpunkt: 225-228 °C

| $C_{22}H_{20}N_2O_3S$ (392,47) | | | | | | |
|---|---|---|---|---|---|---|
| Ber.:<br>Gef.: | C | 67,33<br>67,12 | H | 5,14<br>5,16 | N | 7,14<br>6,95 |

d) 6-(2-(4-Toluolsulfonylamino)indan-5-yl)-6-(3-pyridyl)hex-5-ensäure

5 g 2-(4-Toluolsulfonylamino)indan-5-yl-3-pyridylketon werden zu einer Suspension von 8 g 4-Carboxybutyl-triphenylphosphoniumbromid und 5,6 g Kalium-tert.butylat in 100 ml Tetrahydrofuran unter einer Stickstoffatmosphäre gegeben. Die Suspension wird weitere 2 Stunden bei 0 °C gerührt, dann auf Wasser gegeben und mit Toluol gewaschen. Dann wird die wäßrige Phase mit 3 N Ameisensäure angesäuert und der ausfallende Niederschlag in Methylenchlorid aufgenommen. Die organische Phase wird über Magnesiumsulfat getrocknet und einrotiert. Das erhaltene Öl wird über eine Kieselgelsäule mit Essigsäureethylester als Fließmittel chromatographiert.
Ausbeute: 3,4 g (56 % der Theorie),
Schmelzpunkt: 150-156 °C

| $C_{27}H_{28}N_2O_4S$ (476,59) | | | | | | |
|---|---|---|---|---|---|---|
| Ber.:<br>Gef.: | C | 68,04<br>67,90 | H | 5,92<br>6,10 | N | 5,88<br>5,82 |

Beispiel 8

6-(2-(4-Brombenzolsulfonylamino)indan-5-yl)-6-(3-pyridyl)hex-5-ensäuremethylester

a) 6-(2-Acetylamino-indan-5-yl)-6-(3-pyridyl)hex-5-en-carbonsäure

11,1 g 4-Carboxybutyltriphenylphosphoniumbromid und 8,0 g Kalium-tert.butylat werden in 100 ml absolutem Tetrahydrofuran vorgelegt und bei 10 °C unter einer Stickstoffatmosphäre gerührt. Anschließend gibt man 5,6 g 2-Acetylaminoindan-5-yl-3-pyridylketon portionsweise zu und rührt bei Raumtemperatur 2 Stunden. Danach wird das Reaktionsgemisch auf Eiswasser gegeben und mit Toluol gewaschen. Die wäßrige Phase wird mit 3 N Salzsäure auf pH 5 eingestellt. Der dabei ausfallende Niederschlag wird in Methylenchlorid aufgenommen, mit Wasser gewaschen, über Natriumsulfat getrocknet und einrotiert. Das Produktgemisch wird über eine Kieselgelsäule mit dem Fließmittel Essigsäureethylester:Ethanol:Eisessig (94:5:1) chromatographiert.
Ausbeute: 7,2 g (99 % der Theorie),
Öl, $R_f$-Wert: 0,20 (Kieselgel: Essigsäureethylester/Ethanol/Eisessig = 94:5:1)

b) 6-(2-Aminoindan-5-yl)-6-(3-pyridyl)hex-5-ensäuremethylester

3,1 g 6-(2-Acetylaminoindan-5-yl)6-(3-pyridyl)hex-5-ensäure werden mit 20 ml halbkonzentrierter Salzsäure 15 Stunden am Rückfluß erhitzt und dann einrotiert. Der Rückstand wird anschließend in 50 ml Methanol gegeben, das mit trockenem Chlorwasserstoff gesättigt wird. Nach 30 Minuten Rühren bei Raumtemperatur wird das Reaktionsgemisch zur Trockene einrotiert. Der Rückstand wird in 1 N Natronlauge aufgenommen und auf pH 10 eingestellt. Anschließend wird mit 3 x 50 ml Methylenchlorid extrahiert, die organische Phase getrocknet und einrotiert.
Ausbeute: 2,45 g (50 % der Theorie)
Harz, $R_f$ = 0,50 (Kieselgel: Toluol/Dioxan/Methanol/Ammoniak = 2:5:2:1)

| $C_{21}H_{24}N_2O_2$ (336,44) | | | | | | |
|---|---|---|---|---|---|---|
| Ber.:<br>Gef.: | C | 74,97<br>75,00 | H | 7,19<br>7,01 | N | 8,33<br>8,11 |

c) 6-(2-(4-Brombenzolsulfonylamino)indan-5-yl)-6-(3-pyridyl)hex-5-ensäuremethylester

3,4 g 6-(2-Aminoindan-5-yl)-6-(3-pyridyl)hex-5-ensäuremethylester wird mit 3,3 g 4-Brombenzolsulfonsäurechlorid in 40 ml Chloroform vorgelegt und portionsweise bei Raumtemperatur mit 1,8 g Triethylamin versetzt. Nach 30 Minuten wird die Lösung mit Wasser gewaschen, getrocknet und einrotiert. Das gelbe Öl

wird dann über eine Kieselgelsäule mit Cyclohexan/Essigsäureethylester (1:2) chromatographiert.
Ausbeute: 4,5 g (81 % der Theorie),
Harz, $R_f$-Wert: 0,25 (Kieselgel: Cyclohexan/Essigsäureethylester = 1:1)

| $C_{27}H_{27}BrN_2O_4S$ (555,48) | | | | | | |
|---|---|---|---|---|---|---|
| Ber.:<br>Gef.: | C | 58,38<br>58,30 | H | 4,90<br>5,16 | N | 5,04<br>4,94 |

Analog werden folgende Verbindungen erhalten:
6-(2-(4-Chlorbenzolsulfonylamino)indan-5-yl)-6-(3-pyridyl)hex5-ensäuremethylester.
Harz, $R_f$-Wert: 0,32 (Kieselgel: Cyclohexan/Essigsäureethylester = 1:1)

| $C_{27}H_{27}ClN_2O_4S$ (511,03) | | | | | | |
|---|---|---|---|---|---|---|
| Ber.:<br>Gef.: | C | 63,46<br>63,58 | H | 5,32<br>5,49 | N | 5,48<br>5,35 |

6-(2-(4-Fluorbenzolsulfonylamino)indan-5-yl)-6-(3-pyridyl)hex-5-ensäuremethylester
Harz, $R_f$-Wert: 0,82 (Kieselgel: Toluol/Dioxan/Methanol/Ammoniak = 2:5:2:1)

| $C_{27}H_{27}FN_2O_4S$ (494,58) | | | | | | |
|---|---|---|---|---|---|---|
| Ber.:<br>Gef.: | C | 65,57<br>65,39 | H | 5,50<br>5,78 | N | 5,66<br>5,48 |

6-(2-(2-Thiophensulfonylamino)indan-5-yl)-6-(3-pyridyl)hex-5-ensäuremethylester
Harz, $R_f$-Wert: 0,25 (Kieselgel: Cyclohexan/Essigsäureethylester = 1:1)

| $C_{25}H_{26}N_2O_4S_2$ (482,61) | | | | | | |
|---|---|---|---|---|---|---|
| Ber.:<br>Gef.: | C | 62,22<br>62,28 | H | 5,43<br>5,60 | N | 5,80<br>5,53 |

6-(2-(2,5-Dichlorbenzolsulfonylamino)indan-5-yl)-6-(3-pyridyl)hex-5-ensäuremethylester
Harz, $R_f$-Wert: 0,38 (Kieselgel: Cyclohexan/Essigsäureethylester = 1:1)

| $C_{27}H_{26}Cl_2N_2O_4S$ (545,48) | | | | | | |
|---|---|---|---|---|---|---|
| Ber.:<br>Gef.: | C | 59,45<br>59,41 | H | 4,80<br>5,02 | N | 5,14<br>4,96 |

6-(2-(4-Nitrobenzolsulfonylamino)indan-5-yl)-6-(3-pyridyl)hex-5-ensäuremethylester
Harz, $R_f$-Wert: 0,38 (Kieselgel: Cyclohexan/Essigsäureethylester = 1:1)

| $C_{27}H_{27}N_3O_6S$ (521,59) | | | | | | |
|---|---|---|---|---|---|---|
| Ber.:<br>Gef.: | C | 62,17<br>62,22 | H | 5,22<br>5,45 | N | 8,06<br>7,90 |

6-(2-(Benzolsulfonylamino)indan-5-yl)-6-(3-pyridyl)hex-5-ensäuremethylester
Harz, $R_f$-Wert: 0,20 (Kieselgel: Cyclohexan/Essigsäureethylester = 1:1)

| $C_{27}H_{28}N_2O_4S$ (476,59) | | | | | | |
|---|---|---|---|---|---|---|
| Ber.:<br>Gef.: | C | 68,04<br>67,98 | H | 5,92<br>6,07 | N | 5,88<br>5,60 |

Beispiel 9

6-(2-(4-Brombenzolsulfonylamino)indan-5-yl)-6-(3-pyridyl)hex-5-ensäure

3,7 g 6-(2-(4-Brombenzolsulfonylamino)indan-5-yl)-6-(3-pyridyl)hex-5-ensäuremethylester werden in 30 ml Ethanol und mit 1 ml 15 N Natronlauge 15 Minuten am Rückfluß erhitzt. Die erkaltete Lösung wird einrotiert und der Rückstand in Wasser aufgenommen und mit 30 ml Methylenchlorid gewaschen. Anschließend wird die wässrige Phase mit Salzsäure auf pH 4 eingestellt. Der ausfallende Niederschlag wird gewaschen und getrocknet.
Ausbeute: 3,2 g (88 % der Theorie),
Schmelzpunkt: 83-102 °C

| $C_{25}H_{26}BrN_2O_4S$ (541,46) | | | | | | |
|---|---|---|---|---|---|---|
| Ber.:<br>Gef.: | C | 57,68<br>57,58 | H | 4,65<br>4,64 | N | 5,17<br>4,99 |

Analog werden folgende Verbindungen erhalten:
6-(2-(4-Chlorbenzolsulfonylamino)indan-5-yl)-6-(3-pyridyl)hex-5-ensäure
Schmelzpunkt: 83-98 °C

| $C_{26}H_{25}ClN_2O_4S$ (497,01) | | | | | | |
|---|---|---|---|---|---|---|
| Ber.:<br>Gef.: | C | 62,83<br>62,64 | H | 5,07<br>5,02 | N | 5,64<br>5,57 |

6-(2-(4-Fluorbenzolsulfonylamino)indan-5-yl)-6-(3-pyridyl)hex-5-ensäure
Schmelzpunkt: 73-90 °C

| $C_{26}H_{25}FN_2O_4S$ (480,55) | | | | | | |
|---|---|---|---|---|---|---|
| Ber.:<br>Gef.: | C | 64,98<br>64,85 | H | 5,24<br>5,23 | N | 5,83<br>5,76 |

6-(2-(2-Thiophensulfonylamino)indan-5-yl)-6-(3-pyridyl)hex-5-ensäure
Schmelzpunkt: 67-90 °C

| $C_{24}H_{24}N_2O_4S_2$ (468,58) | | | | | | |
|---|---|---|---|---|---|---|
| Ber.:<br>Gef.: | C | 61,52<br>61,38 | H | 5,16<br>5,04 | N | 5,98<br>5,70 |

6-(2-Benzolsulfonylamino)indan-5-yl)-6-(3-pyridyl)hex-5-ensäure
Schmelzpunkt: 70-97 °C

| $C_{26}H_{26}N_2O_4S$ (462,58) | | | | | | |
|---|---|---|---|---|---|---|
| Ber.:<br>Gef.: | C | 67,51<br>67,44 | H | 5,67<br>5,87 | N | 6,06<br>6,18 |

6-(2-(4-Nitrobenzolsulfonylamino)indan-5-yl)-6-(3-pyridyl)hex-5-ensäure
Schmelzpunkt: 73-92 °C

| $C_{26}H_{25}N_3O_6S$ (507,56) | | | | | | |
|---|---|---|---|---|---|---|
| Ber.:<br>Gef.: | C | 61,53<br>61,45 | H | 4,96<br>5,06 | N | 8,28<br>8,18 |

6-(2-(2,5-Dichlorbenzolsulfonylamino)indan-5-yl)-6-(3-pyridyl)hex-5-ensäure
Schmelzpunkt: 79-100 °C

| $C_{26}H_{24}Cl_2N_2O_4S$ (531,45) | | | | | | |
|---|---|---|---|---|---|---|
| Ber.:<br>Gef.: | C | 58,76<br>58,78 | H | 4,55<br>4,65 | N | 5,27<br>5,11 |

Beispiel 10

6-(4-(2-(4-Chlorbenzolsulfonylaminoethyl)phenyl-6-(3-pyridyl)hexansäure

a) 4-(2-Acetylaminoethyl)phenyl-3-pyridylketon

180 g Aluminiumchlorid werden mit 35 ml Dimethylformamid versetzt, wobei die Temperatur auf 70 °C steigt. Anschließend gibt man 66,8 g Nicotinsäurechlorid-hydrochlorid und dann 49 g 2-Acetylaminoethylbenzol bei 70 °C zu. Nach 2 Stunden wird abgekühlt und mit 60 ml Ethylenchlorid versetzt. Dann gießt man den Ansatz auf Eiswasser und 180 ml konzentrierter Salzsäure. Die wässerige Phase wird mit Natronlauge alkalisch gestellt und dann mit 3 x 100 ml Ethylenchlorid extrahiert. Die organische Phase wird getrocknet und einrotiert.
Ausbeute: 70,4 g (87 % der Theorie),
Öl, $R_f$-Wert: 0,47 (Kieselgel: Ethylenchlorid/Methanol = 9:1)

| $C_{16}H_{16}N_2O_2$ (268,32) | | | | | | |
|---|---|---|---|---|---|---|
| Ber.:<br>Gef.: | C | 71,62<br>71,82 | H | 6,01<br>6,20 | N | 10,44<br>10,30 |

b) 6-(4-(2-Acetylaminoethyl)phenyl)-6-(3-pyridyl)hex-5-ensäure

Hergestellt analog Beispiel 7d aus 4-(2-Acetylaminoethyl)phenyl-3-pyridylketon und 4-Carboxybutyltriphenylphosphoniumbromid.
Ausbeute: 57 % der Theorie,
Schmelzpunkt: 80-85 °C

| $C_{21}H_{24}N_2O_3$ (352,4) | | | | | | |
|---|---|---|---|---|---|---|
| Ber.:<br>Gef.: | C | 71,57<br>71,23 | H | 6,86<br>7,06 | N | 7,95<br>7,94 |

Analog werden folgende Verbindungen erhalten:
5-(4-(2-Acetylaminoethyl)phenyl)-5-(3-pyridyl)pent-4-ensäure
Ausbeute: 87 % der Theorie,
Harz, $R_f$-Wert: 0,35 (Kieselgel: Chloroform/Methanol = 10:1)

| $C_{20}H_{22}N_2O_3$ (338,4) | | | | | | |
|---|---|---|---|---|---|---|
| Ber.:<br>Gef.: | C | 70,98<br>70,79 | H | 6,55<br>6,39 | N | 8,28<br>7,88 |

7-(4-(2-Acetylaminoethyl)phenyl)-7-(3-pyridyl)hept-6-ensäure
Ausbeute: 49 % der Theorie,
Harz, $R_f$-Wert: 0,37 (Kieselgel: Chloroform/Methanol = 10:1)

| $C_{22}H_{26}N_2O_3$ (366,5) | | | | | | |
|---|---|---|---|---|---|---|
| Ber.:<br>Gef.: | C | 72,11<br>71,82 | H | 7,15<br>7,41 | N | 7,64<br>7,58 |

c) 6-(4-(2-Acetylaminoethyl)phenyl)-6-(3-pyridyl)hexansäure

7,05 g 6-(4-(2-Acetylaminoethyl)phenyl)-6-(3-pyridyl)hex-5-ensäure werden in 85 ml 0,7 N Natronlauge gelöst und bei 40 °C mit 1 g Palladium/Kohle katalytisch reduziert. Nach Absaugen des Katalysators wird auf pH 6 angesäuert und das ausfallende Öl in Essigsäureethylester aufgenommen und eingeengt. Das Rohprodukt wird aus Methanol umkristallisiert.
Ausbeute: 4,7 g (66 % der Theorie),
Schmelzpunkt: 135-139 °C

| $C_{21}H_{26}N_2O_2$ (354,5) | | | | | | |
|---|---|---|---|---|---|---|
| Ber.:<br>Gef.: | C | 71,16<br>70,85 | H | 7,39<br>7,50 | N | 7,90<br>7,85 |

Analog werden folgende Verbindungen erhalten:
5-(4-(2-Acetylaminoethyl)phenyl)-5-(3-pyridyl)pentansäure
Ausbeute: 58 % der Theorie,
Harz, $R_f$-Wert: 0,37 (Kieselgel: Chloroform/Methanol = 10:1)

| $C_{20}H_{24}N_2O_3$ (340,4) | | | | | | |
|---|---|---|---|---|---|---|
| Ber.:<br>Gef.: | C | 70,56<br>70,40 | H | 7,11<br>6,97 | N | 8,23<br>7,94 |

7-(4-(2-Acetylaminoethyl)phenyl)-7-(3-pyridyl)heptansäure
Ausbeute: 98 % der Theorie,
Harz, $R_f$-Wert: 0,43 (Kieselgel: Chloroform/Methanol = 10:1)

| $C_{22}H_{28}N_2O_3$ (368,5) | | | | | | |
|---|---|---|---|---|---|---|
| Ber.:<br>Gef.: | C | 71,71<br>71,58 | H | 7,66<br>7,77 | N | 7,60<br>7,22 |

d) 6-(4-(2-Aminoethyl)phenyl)-6-(3-pyridyl)hexansäure

4,0 g 6-(4-(2-Acetylaminoethyl)phenyl)-6-(3-pyridyl)hexansäure werden 18 Stunden mit 50 ml halbkonzentrierter Salzsäure am Rückfluß gekocht. Anschließend wird einrotiert und der Rückstand über eine Kieselgelsäule mit Methanol chromatographiert.
Ausbeute: 2,3 g (66 % der Theorie),
Harz, $R_f$-Wert: 0,27 (Kieselgel: Methanol)

| $C_{19}H_{24}N_2O_2$ (312,4) | | | | | | |
|---|---|---|---|---|---|---|
| Ber.:<br>Gef.: | C | 73,05<br>72,81 | H | 7,74<br>7,63 | N | 8,97<br>8,83 |

Analog werden folgende Verbindungen erhalten:
5-(4-(2-Aminoethyl)phenyl)-5-(3-pyridyl)pentansäure
Ausbeute: 96 % der Theorie,
Harz, $R_f$-Wert: 0,33 (Kieselgel: Methanol)

| $C_{18}H_{22}N_2O_2$ x 0.5 HCl (317,1) | | | | | | |
|---|---|---|---|---|---|---|
| Ber.:<br>Gef.: | C | 68,17<br>68,27 | H | 7,43<br>7,31 | N | 9,39<br>8,99 |

7-(4-(2-Aminoethyl)phenyl)-7-(3-pyridyl)heptansäure
Ausbeute: 96 % der Theorie,
Harz, $R_f$-Wert: 0,59 (Kieselgel: Methanol)

| $C_{20}H_{26}N_2O_2$ (326,4) | | | | | | |
|---|---|---|---|---|---|---|
| Ber.:<br>Gef.: | C | 73,59<br>73,48 | H | 8,03<br>8,00 | N | 8,58<br>8,37 |

e) 6-(4-(2-(4-Chlorbenzolsulfonylamino)ethyl)phenyl)-6-(3-pyridyl)hexansäure

1,9 g 6-(4-(2-Aminoethyl)phenyl)-6-(3-pyridyl)-hexansäure werden in 150 ml Dioxan suspendiert und mit 20 ml 5%iger Kaliumcarbonatlösung versetzt. Zu dieser Mischung gibt man bei Raumtemperatur 1,54 g 4-Chlorbenzolsulfonsäurechlorid in 20 ml Dioxan. Nach 5 Stunden wird zur Trockene einrotiert, der Rückstand in wenig Natronlauge aufgenommen und dann mit verdünnter Essigsäure gefällt. Der Niederschlag wird gesammelt, getrocknet und dann über eine Kieselgelsäule mit dem Fließmittel Chloroform/Methanol (10:1) chromatographiert.
Ausbeute: 1,8 g (61 % der Theorie),
Harz, $R_f$-Wert: 0,48 (Kieselgel: Chloroform/Methanol = 10:1)

| $C_{25}H_{27}ClN_2O_4S$ (487,03) | | | | | | |
|---|---|---|---|---|---|---|
| Ber.:<br>Gef.: | C | 61,65<br>61,59 | H | 5,59<br>5,40 | N | 5,79<br>5,74 |

Analog werden folgende Verbindungen erhalten:
6-(4-(2-(4-Fluorbenzolsulfonylamino)ethyl)phenyl)-6-(3-pyridyl)hexansäure
Ausbeute: 11 % der Theorie,
Harz, $R_f$-Wert: 0,53 (Kieselgel: Chloroform/Methanol = 10:1)

| $C_{25}H_{27}FN_2O_4S$ (470,60) | | | | | | |
|---|---|---|---|---|---|---|
| Ber.:<br>Gef.: | C | 63,81<br>63,75 | H | 5,78<br>5,92 | N | 5,95<br>5,80 |

6-(4-(2-(4-Toluolsulfonylamino)ethyl)phenyl)-6-(3-pyridyl)hexansäure
Ausbeute: 13 % der Theorie,
Harz, $R_f$-Wert: 0,55 (Kieselgel: Chloroform/Methanol = 10:1)

| $C_{26}H_{29}N_2O_4S$ (466,60) | | | | | | |
|---|---|---|---|---|---|---|
| Ber.:<br>Gef.: | C | 66,93<br>66,81 | H | 6,48<br>6,57 | N | 6,00<br>5,94 |

6-(4-(2-(4-Brombenzolsulfonylamino)ethyl)phenyl)-6-(3-pyridyl)hexansäure
Ausbeute: 24 % der Theorie,
Harz, $R_f$-Wert: 0,34 (Kieselgel: Chloroform/Methanol = 20:1)

| $C_{25}H_{27}BrN_2O_4S$ (531,50) | | | | | | |
|---|---|---|---|---|---|---|
| Ber.:<br>Gef.: | C | 56,50<br>56,41 | H | 5,12<br>5,31 | N | 5,27<br>5,17 |

Beispiel 11

5-(4-(2-(4-Fluorbenzolsulfonylamino)ethyl)phenyl)-5-(3-pyridyl)pentansäure

Hergestellt analog Beispiel 10e aus 5-(4-(2-Aminoethyl)phenyl)-5-(3-pyridyl)pentansäure und 4-Fluorbenzolsulfonsäurechlorid.
Ausbeute: 28 % der Theorie,
Harz, $R_f$-Wert: 0,33 (Kieselgel: Chloroform/Methanol = 10:1)

| $C_{24}H_{25}FN_2O_4S$ (456,50) | | | | | | |
|---|---|---|---|---|---|---|
| Ber.:<br>Gef.: | C | 63,14<br>63,04 | H | 5,52<br>5,60 | N | 6,94<br>5,96 |

Beispiel 12

5-(4-(2-(4-Chlorbenzolsulfonylamino)ethyl)phenyl)-5-(3-pyridyl)pentansäure

Hergestellt analog Beispiel 10e aus 5-(4-(2-Aminoethyl)phenyl)-5-(3-pyridyl)pentansäure und 4-Chlorbenzolsulfonsäurechlorid.
Ausbeute: 21 % der Theorie,
Schmelzpunkt: 70 °C

| $C_{24}H_{25}ClN_2O_4S$ (473,00) | | | | | | |
|---|---|---|---|---|---|---|
| Ber.:<br>Gef.: | C | 60,94<br>61,01 | H | 5,33<br>5,35 | N | 5,92<br>5,70 |

Beispiel 13

7-(4-(2-(4-Toluolbenzolsulfonylamino)ethyl)phenyl)-7-(3-pyridyl)heptansäure

Hergestellt analog Beispiel 10e aus 7-(4-(2-Aminoethyl)-phenyl)-7-(3-pyridyl)heptansäure und 4-Toluolsulfonsäurechlorid.
Ausbeute: 78 % der Theorie,
Harz, $R_f$-Wert: 0,42 (Kieselgel: Chloroform/Methanol = 10:1)

| $C_{27}H_{32}N_2O_4S$ (480,60) | | | | | | |
|---|---|---|---|---|---|---|
| Ber.:<br>Gef.: | C | 67,47<br>67,34 | H | 6,71<br>6,71 | N | 5,83<br>5,74 |

Beispiel 14

7-(4-(2-(4-Fluorbenzolsulfonylamino)ethyl)phenyl)-7-(3-pyridyl)heptansäure

Hergestellt analog Beispiel 13 aus 7-(4-(2-Aminoethyl)phenyl)-7-(3-pyridyl)heptansäure und 4-Fluorbenzolsulfonsäurechlorid.
Ausbeute: 66 % der Theorie,
Harz, $R_f$-Wert: 0,20 (Kieselgel: Chloroform/Methanol = 10:1)

| $C_{26}H_{29}FN_2O_4S$ (484,6) | | | | | | |
|---|---|---|---|---|---|---|
| Ber.:<br>Gef: | C | 64,44<br>64,48 | H | 6,03<br>5,99 | N | 5,72<br>5,72 |

Beispiel 15

5-(4-(2-(4-Chlorbenzolsulfonylamino)ethyl)phenyl)-5-(3-pyridyl)pentansäuremethylester

2,0 g 5-(4-(2-(4-Chlorbenzolsulfonylamino)ethyl)phenyl)-5-(3-pyridyl)pentansäure werden in 30 ml Methanol gelöst und mit 3 ml Thionylchlorid bei 0 °C versetzt. Die Lösung wird über Nacht gerührt, dann einrotiert und der Rückstand über eine Kieselgelsäule chromatographiert.
Ausbeute: 1,1 g (53 % der Theorie),
Harz, $R_f$-Wert: 0,65 (Kieselgel: Chloroform/Methanol = 95:5)

| $C_{26}H_{29}ClN_2O_4S$ (501,1) | | | | | | |
|---|---|---|---|---|---|---|
| Ber.:<br>Gef.: | C | 62,33<br>62,36 | H | 5,83<br>6,01 | N | 5,59<br>5,42 |

Beispiel 16

5-(2-(4-Chlorbenzolsulfonylamino)indan-5-yl)-5-(3-pyridyl)pent-4-ensäuremethylester

3,7 g 5-(2-(4-Chlorbenzolsulfonylamino)indan-5-yl)-5-(3-pyridyl)pent-4-ensäure werden in 30 ml Methanol gelöst, in die getrockneter Chlorwasserstoff eingeleitet wird. Die Lösung wird über Nacht gerührt und dann einrotiert. Unter Eiskühlung wird mit wässeriger Kaliumcarbonyltlösung die Base freigesetzt, die mit Methylenchlorid extrahiert wird. Die Lösung wird einrotiert und der Rückstand über eine Kieselgelsäule chromatographiert.
Ausbeute: 2,5 g (52 % der Theorie),
Harz, $R_f$-Wert: 0,53 (Kieselgel: Toluol/Dioxan/Ethanol/Essigsäure = 9:1:1:0,6)

| $C_{26}H_{25}ClN_2O_4S$ (497,01) | | | | | | |
|---|---|---|---|---|---|---|
| Ber.:<br>Gef: | C | 62,80<br>62,67 | H | 5,10<br>5,39 | N | 5,60<br>5,40 |

Analog wird folgende Verbindung erhalten:
7-(2-(4-Chlorbenzolsulfonylamino)indan-5-yl)-7-(3-pyridyl)hept-6-ensäuremethylester
Ausbeute: 73 % der Theorie,
Harz, $R_f$-Wert: 0,31 (Kieselgel: Cyclohexan/Essigsäureethylester = 1:1)

| $C_{28}H_{29}ClN_2O_4S$ (525,06) | | | | | | |
|---|---|---|---|---|---|---|
| Ber.:<br>Gef: | C | 64,05<br>64,45 | H | 5,56<br>6,15 | N | 5,33<br>5,05 |

Beispiel 17

5-(2-(4-Chlorbenzolsulfonylamino)indan-5-yl)-5-(3-pyridyl)pent-4-ensäure

Hergestellt aus 5-(2-(4-Chlorbenzolsulfonylamino)indan-5-yl)5-(3-pyridyl)pent-4-ensäuremethylester durch Hydrolyse mit Natronlauge.
Ausbeute: 95 % der Theorie,
Schmelzpunkt: 94-114 °C

| $C_{25}H_{23}ClN_2O_4S$ (482,98) | | | | | | |
|---|---|---|---|---|---|---|
| Ber.:<br>Gef: | C | 62,20<br>62,14 | H | 4,80<br>4,70 | N | 5,80<br>5,81 |

Analog wird folgende Verbindung erhalten:
7-(2-(4-Chlorbenzolsulfonylamino)indan-5-yl)-7-(3-pyridyl)hept-6-ensäure
Ausbeute: 94 % der Theorie,
Schmelzpunkt: 66-90 °C

| $C_{27}H_{27}ClN_2O_4S$ (511,03) | | | | | | |
|---|---|---|---|---|---|---|
| Ber.:<br>Gef: | C | 63,50<br>63,65 | H | 5,30<br>5,29 | N | 5,50<br>5,30 |

Beispiel 18

(Z)- und (E)-6-(2-(4-Chlorbenzolsulfonylamino)indan-5-yl)-6-(3-pyridyl)hex-5-ensäure

1,9 g 6-(2-(4-Chlorbenzolsulfonylamino)indan-5-yl)-6-(3-pyridyl)hex-5-ensäuremethylester werden über eine Kieselgelsäule mit dem Fließmittel Ethylenchlorid/Essigsäureethylester/Eisessig (70:30:5) chromatographiert. Die schneller laufende Substanz ist das Z-Isomere. Die so erhaltenen (Z)- und (E)-Ester werden analog Beispiel 17 mit Natronlauge hydrolisiert.
(Z)-6-(2-(4-Chlorbenzolsulfonylamino)indan-5-yl)-6-(3-pyridyl)-hex-5-ensäure
Ausbeute: 200 mg (10 % der Theorie),
Schmelzpunkt: 70-100 °C

| $C_{26}H_{25}ClN_2O_4S$ (497,01) | | | | | | |
|---|---|---|---|---|---|---|
| Ber.:<br>Gef: | C | 62,83<br>62,72 | H | 5,07<br>5,24 | N | 5,64<br>5,47 |

(E)-6-(2-(4-Chlorbenzolsulfonylamino)indan-5-yl)-6-(3-pyridyl)-hex-5-ensäure
Ausbeute: 400 mg (20 % der Theorie),
Schmelzpunkt: 75-103 °C

| $C_{26}H_{25}ClN_2O_4S$ (497,01) | | | | | | |
|---|---|---|---|---|---|---|
| Ber.:<br>Gef: | C | 62,83<br>62,75 | H | 5,07<br>5,14 | N | 5,64<br>5,43 |

Beispiel 19

6-(2-(4-Chlorbenzolsulfonylamino)indan-5-yl)-6-(3-pyridyl)hexansäure

3,0 g 6-(2-(4-Chlorbenzolsulfonylamino)indan-5-yl)-6-(3-pyridyl)hex-5-ensäure werden in 50 ml 0,3 N Natronlauge gelöst und mit 1 g Palladium/Kohle bei 40 °C und 3,5 bar 12 Stunden hydriert. Dann wird der Katalysator abgesaugt und das Filtrat auf pH 4-5 eingestellt. Das ausfallende Produkt wird abgetrennt und in Chloroform aufgenommen. Der organische Extrakt wird mit Wasser gewaschen, getrocknet und eingeengt. Anschließend wird das Gemisch über eine Kieselgelsäule mit dem Fließmittel Ethylenchlorid/Essigsäureethylester/Eisessig = (70:30:2) chromatographiert. Die dritte Fraktion enthält das gewünschte Produkt.
Ausbeute: 0,4 g (13 % der Theorie),
Schmelzpunkt: 85-100 °C

| $C_{26}H_{27}ClN_2O_4S$ | | | | | | |
|---|---|---|---|---|---|---|
| Ber.:<br>Gef: | C | 62,58<br>62,54 | H | 5,45<br>5,45 | N | 5,61<br>5,79 |

Beispiel 20

7-(2-(4-Chlorbenzolsulfonylamino)indan-5-yl)-7-(3-pyridyl)heptansäure

Hergestellt analog Beispiel 19 durch Reduktion von 7-(2-(4Chlorbenzolsulfonylamino)indan-5-yl)-7-(3-pyridyl)hept-6-ensäure mit Platin/Kohle.
Ausbeute: 40 % der Theorie,
Harz, $R_f$-Wert: 0,3 (Kieselgel: Ethylenchlorid/Essigsäureethylester/Essigsäure = 10:3:0,5)

| $C_{26}H_{29}ClN_2O_4S$ (501,03) | | | | | | |
|---|---|---|---|---|---|---|
| Ber.:<br>Gef: | C | 62,32<br>62,56 | H | 5,83<br>6,00 | N | 5,59<br>5,32 |

Beispiel 21

5-(2-(Benzolsulfonylamino)indan-5-yl)-5-(3-pyridyl)pentansäure

Hergestellt aus 5-(2-(4-Chlorbenzolsulfonylamino)indan-5-yl)-5-(3-pyridyl)pent-4-ensäure analog Beispiel 19 durch katalytische Hydrierung in Gegenwart von Platin als Katalysator.

Ausbeute: 37 % der Theorie,
Schmelzpunkt: 80-110 ° C

| $C_{25}H_{25}ClN_2O_4S$ (485,00) | | | | | | |
|---|---|---|---|---|---|---|
| Ber.:<br>Gef: | C | 61,91<br>61,85 | H | 5,19<br>5,33 | N | 5,77<br>6,05 |

Beispiel 22

7-(2-(Benzolsulfonylamino)indan-5-yl)-7-(3-pyridyl)heptansäure

Hergestellt aus 7-(2-(4-Chlorbenzolsulfonylamino)indan-5-yl)-7-(3-pyridyl)hept-6-ensäure durch katalytische Hydrierung analog Beispiel 21.
Ausbeute: 10 % der Theorie,
Schmelzpunkt: 60-75 ° C

| $C_{27}H_{29}ClN_2O_4S$ (513,05) | | | | | | |
|---|---|---|---|---|---|---|
| Ber.:<br>Gef: | C | 63,21<br>63,43 | H | 5,69<br>5,88 | N | 5,46<br>5,63 |

Beispiel 23

3-(2-(4-Toluolsulfonylamino)indan-5-yl)-3-(3-pyridyl)prop-2-ensäure

a) 3-(2-(4-Toluolsulfonylamino)indan-5-yl)-3-(3-pyridyl)prop-2-ensäureethylester

Zu einer Suspension von 9,6 g Kalium-tert.butylat in 100 ml Tetrahydrofuran und 25 ml Dimethylformamid werden bei 5 ° C 9,84 g Phosphonoessigsäuretriethylester gegeben. Nach 30 Minuten Rühren bei 0 ° C gibt man 15,5 g 2-(4-Toluolsulfonylamino)indan-5-yl-3-pyridylketon zu. Anschließend wird 5 Stunden am Rückfluß gekocht. Die Lösung wird auf Eiswasser gegeben und mit 4 x 50 ml Methylenchlorid extrahiert. Dann wird getrocknet, einrotiert und der Rückstand über eine Kieselgelsäule mit dem Fließmittel Ethylenchlorid/Essigsäureethylester (9:1) chromatographiert.
Ausbeute: 17,2 g (93 % der Theorie),
$R_f$-Wert: 0,46/0,35 (Kieselgel: Ethylenchlorid/Essigsäureethylester = 1:1)

b) 3-(2-(4-Toluolsulfonylamino)indan-5-yl)-3-(3-pyridyl)prop-2-ensäure

4,2 g 3-(2-(4-Toluolsulfonylamino)indan-5-yl)-3-(3-pyridyl)prop-2-ensäureethylester werden in 40 ml Ethanol und 1,5 ml 15 N Natronlauge 30 Minuten unter Rückfluß gekocht. Anschließend wird die erkaltete Lösung mit 3 x 50 ml Methylenchlorid gewaschen und dann angesäuert. Der ausgefallene Niederschlag wird gewaschen, getrocknet und anschließend aus n-Butanol umkristallisiert.
Ausbeute: 2,3 g (58 % der Theorie),
Schmelzpunkt: 228-230 ° C

| $C_{23}H_{22}N_2O_4S$ (422,5) | | | | | | |
|---|---|---|---|---|---|---|
| Ber.:<br>Gef: | C | 65,38<br>65,32 | H | 5,24<br>5,17 | N | 6,63<br>6,48 |

Beispiel 24

3-(2-(4-(Toluolsulfonylamino)indan-5-yl)-3-(3-pyridyl)propansäure

Hergestellt analog Beispiel 19 aus 3-(2-(4-Toluolsulfonylamino)indan-5-yl)-3-(3-pyridyl)prop-2-ensäure und anschließendes Fällen aus Dioxan durch Zugabe von Diisopropylether.
Ausbeute: 74 % der Theorie,
Schmelzpunkt: 85-97 °C

| $C_{23}H_{22}N_2O_4S$ x 0,8 Dioxan (424,51) | | | | | | |
|---|---|---|---|---|---|---|
| Ber.:<br>Gef: | C | 63,57<br>63,39 | H | 6,19<br>6,30 | N | 5,66<br>5,62 |

Beispiel 25

6-(4-(2-(4-Fluorbenzolsulfonylamino)ethyl)phenyl)-6-(3-pyridyl)-hex-5-ensäure

3,1 g 6-(4-(2-Aminoethyl)phenyl)-6-(3-pyridyl)hex-5-ensäure (hergestellt analog Beispiel 10d) werden in 150 ml Dioxan mit 5 ml gesättigter Kaliumcarbonatlösung gerührt. Anschließend gibt man 2,9 g 4-Fluorbenzolsulfonsäurechlorid in 20 ml Dioxan zu und rührt bei Raumtemperatur über Nacht. Anschließend wird mit Essigsäure versetzt, wobei ein Niederschlag ausfällt. Dieser wird abgetrennt und in Essigsäureethylester aufgenommen, getrocknet und eingeengt. Schließlich wird der Rückstand über eine Kieselgelsäule mit dem Fließmittel Chloroform/Methanol (20:1) chromatographiert.
Ausbeute: 0,5 g (10 % der Theorie),
Harz, $R_f$-Wert: 0,55 (Kieselgel: Chloroform/Methanol = 10:1)

| $C_{25}H_{25}FN_2O_4S$ (470,6) | | | | | | |
|---|---|---|---|---|---|---|
| Ber.:<br>Gef: | C | 64,09<br>63,77 | H | 5,38<br>5,59 | N | 5,98<br>6,00 |

Analog werden folgende Verbindungen erhalten:
6-(4-(2-(4-Toluolsulfonylamino)ethyl)phenyl)-6-(3-pyridyl)hex-5-ensäure
Ausbeute: 13% der Theorie,
Harz, $R_f$-Wert: 0,5 (Kieselgel: Chloroform/Methanol = 10:1)

| $C_{26}H_{28}N_2O_4S$ (464,6) | | | | | | |
|---|---|---|---|---|---|---|
| Ber.:<br>Gef: | C | 67,22<br>67,06 | H | 6,07<br>6,21 | N | 6,03<br>5,86 |

6-(4-(2-(4-Brombenzolsulfonylamino)ethyl)phenyl)-6-(3-pyridyl)-hex-5-ensäure
Ausbeute: 20 % der Theorie,
Harz, $R_f$-Wert: 0,4 (Kieselgel: Chloroform/Methanol = 10:1)

| $C_{25}H_{25}BrN_2O_4S$ (529,5) | | | | | | |
|---|---|---|---|---|---|---|
| Ber.:<br>Gef: | C | 56,71<br>56,72 | H | 4,76<br>4,58 | N | 5,29<br>5,12 |

Beispiel 26

7-(4-(2-(4-Chlorbenzolsulfonylamino)ethyl)phenyl)-7-(3-pyridyl)-hept-6-ensäure

Hergestellt aus 4-(2-(4-Chlorbenzolsulfonylamino)ethyl)phenyl-3-pyridylketon analog Beispiel 7d.
Ausbeute: 83 % der Theorie,
Harz, $R_f$-Wert: 0,5 (Kieselgel: Ethylenchlorid/Methanol = 5:1)

| $C_{26}H_{27}ClN_2O_4S$ (499,02) | | | | | | |
|---|---|---|---|---|---|---|
| Ber.:<br>Gef.: | C | 62,58<br>62,48 | H | 5,45<br>5,40 | N | 5,62<br>5,62 |

Beispiel 27

6-(4-(2-(4-Chlorbenzolsulfonylamino)ethyl)phenyl)-6-(3-pyridyl)-hex-5-ensäurediethylamid

1,0 g 6-(4-(2-(4-Chlorbenzolsulfonylamino)ethyl)phenyl)-6-(3-pyridyl)hex-5-ensäure werden in 15 ml Tetrahydrofuran gelöst und mit 0,49 g Carbonyldiimidazol 15 Minuten gerührt. Anschließend gibt man 1 ml Diethylamin zu und erhitzt 2 Stunden am Rückfluß. Dann wird eingeengt und der Rückstand in Essigester aufgenommen und getrocknet. Schließlich wird über eine Kieselgelsäule chromatographiert mit dem Fließmittel Essigsäureethylester.
Ausbeute: 0,6 g (54 % der Theorie),
Öl, $R_f$-Wert: 0,23 (Kieselgel: Essigsäureethylester)

| $C_{29}H_{34}ClN_3O_3S$ (540,12) | | | | | | |
|---|---|---|---|---|---|---|
| Ber.:<br>Gef.: | C | 64,48<br>64,42 | H | 6,34<br>6,60 | N | 7,78<br>7,52 |

Analog wird folgende Verbindung erhalten:
6-(4-(2-(4-Chlorbenzolsulfonylamino)ethyl)phenyl)-6-(3-pyridyl)-hex-5-ensäurebenzylamid
Ausbeute: 51 % der Theorie,
Harz, $R_f$-Wert: 0,37 (Kieselgel: Essigsäureethylester)

| $C_{32}H_{32}ClN_3O_3S$ (574,14) | | | | | | |
|---|---|---|---|---|---|---|
| Ber.:<br>Gef.: | C | 66,94<br>66,72 | H | 5,61<br>5,44 | N | 7,31<br>7,10 |

Beispiel 28

6-(4-(2-(N-Methyl-4-chlorbenzolsulfonylamino)ethyl)phenyl)-6-(3-pyridyl)hex-5-ensäure

2,0 g 6-(4-(2-(4-Chlorbenzolsulfonylamino)ethyl)-phenyl)-6-(3-pyridyl)hex-5-ensäure werden in 10 ml 4 N Natronlauge, 100 ml Methylenchlorid, 80 mg Benzyltrimethylammoniumchlorid und 0,85 g Methyljodid über Nacht gerührt. Anschließend wird die organische Phase abgetrennt und die Wasserphase auf pH 5 angesäuert. Das ausgefallene Produkt wird abgetrennt und in Methylenchlorid aufgenommen, getrocknet und eingeengt. Schließlich wird der Rückstand mit Ethylenchlorid/Methanol (97:3) über eine Kieselgelsäule chromatographiert.
Ausbeute: 0,43 g (21 % der Theorie),
Schmelzpunkt: 121-125 °C

| $C_{26}H_{27}ClN_2O_4S$ (499,02) | | | | | | |
|---|---|---|---|---|---|---|
| Ber.:<br>Gef: | C | 62,58<br>62,53 | H | 5,45<br>5,54 | N | 5,61<br>5,53 |

Beispiel 29

6-(4-(2-(4-Chlorbenzolsulfonylamino)ethyl)phenyl)-6-(3-pyridyl)-2,2-dimethyl-hex-5-ensäure

a) 6-(4-(2-(4-Chlorbenzolsulfonylamino)ethyl)phenyl)-6-(3-pyridyl)-2,2-dimethyl-hex-5-ensäurepiperidid

Hergestellt analog Beispiel 7d aus 4-Triphenylphosphoniumbutansäure-piperidid-bromid und 4-(2-(4-Chlorbenzolsulfonylamino)ethyl)phenyl-3-pyridylketon.
Ausbeute: 6,7 % der Theorie.
Harz, $R_f$-Wert: 0,4 (Kieselgel: Essigsäureethylester)

| $C_{32}H_{38}ClN_3O_3S$ (580,16) | | | | | | |
|---|---|---|---|---|---|---|
| Ber.:<br>Gef: | C | 66,24<br>66,15 | H | 6,60<br>6,33 | N | 7,24<br>7,11 |

b) 6-(4-(2-(4-Chlorbenzolsulfonylamino)ethyl)phenyl)-6-(3-pyridyl)-2,2-dimethyl-hex-5-ensäure

0,35 g 6-(4-(2-(4-Chlorbenzolsulfonylamino)ethyl)phenyl)-6-(3-pyridyl)-2,2-dimethyl-hex-5-ensäurepiperidid werden in 20 ml 6 N Salzsäure 8 Stunden am Rückfluß gekocht. Dann wird eingeengt und der Rückstand in Natronlauge gelöst und mit Salzsäure auf pH 4 eingestellt. Der ausgefallene Niederschlag wird abgesaugt und über eine Kieselgelsäule mit Ethylenchlorid/Methanol (10:1) chromatographiert.
Ausbeute: 0,12 g (39 % der Theorie),
Harz, $R_f$-Wert: 0,5 (Kieselgel: Ethylenchlorid/Methanol = 9:1)

| $C_{27}H_{29}ClN_2O_4S$ (513,04) | | | | | | |
|---|---|---|---|---|---|---|
| Ber.:<br>Gef: | C | 63,21<br>63,08 | H | 5,70<br>5,58 | N | 5,46<br>5,60 |

Beispiel 30

6-(4-(2-(2,4,6-Trimethylbenzolsulfonylamino)ethyl)phenyl)-6-(3-pyridyl)hex-5-ensäure

a) 6-(4-(2-Aminoethyl)phenyl)-6-(3-pyridyl)hex-5-ensäuremethylester

Hergestellt durch Hydrolyse von 6-(4-(2-Acetylaminoethyl)phenyl)-6-(3-pyridyl)hex-5-ensäure und anschließende Veresterung mit Methanol analog Beispiel 8b.
Ausbeute: 87 % der Theorie,
Harz, $R_f$-Wert: 0,6 (Kieselgel: Dioxan/Toluol/Methanol/Ammoniak = 5:2:2:1)

| $C_{20}H_{24}N_2O_2$ (324,42) | | | | | | |
|---|---|---|---|---|---|---|
| Ber.:<br>Gef: | C | 74,05<br>73,85 | H | 7,46<br>7,58 | N | 8,63<br>8,52 |

b) 6-(4-(2-(2,4,6-Trimethylbenzolsulfonylamino)ethyl)phenyl)-6-(3-pyridyl)hex-5-ensäure

Eine Mischung aus 3,24 g 6-(4-(2-Aminoethyl)phenyl)-6-(3-pyridyl)hex-5-ensäuremethylester, 2,2 g 2,4,6-Trimethylbenzolsulfonsäurechlorid und 100 ml Triethylamin in 50 ml Dichlormethan wird 30 Minuten bei Raumtemperatur gerührt. Anschließend wird die Reaktionsmischung zweimal mit Wasser gewaschen, getrocknet und eingeengt. Der Rückstand wird über eine Kieselgelsäule mit Essigsäureethylester gereinigt. Das erhaltene Rohprodukt wird in einer Mischung aus 32 ml Ethanol und 5 ml 4N Natronlauge 30 Minuten auf 50-60 °C erwärmt. Die Reaktionsmischung wird eingeengt, der Rückstand in 50 ml Wasser aufgenommen und mit Essigsäureethylester gewaschen. Die Wasserphase wird durch Zugabe von Zitronensäure auf pH 5 eingestellt und zweimal mit Essigsäureethylester extrahiert. Die organische Phase wird getrocknet, eingeengt und der Rückstand über eine Kieselgelsäule mit Essigsäureethylester chromatographiert. Das Rohprodukt wird anschließend aus Essigsäureethylester/Diisopropylether umkristallisiert.
Ausbeute: 1,35 g (28 % der Theorie),
Schmelzpunkt: 79-83 °C

| $C_{28}H_{32}N_2O_4S$ (492,64) | | | | | | |
|---|---|---|---|---|---|---|
| Ber.:<br>Gef: | C | 68,27<br>68,00 | H | 6,55<br>6,51 | N | 5,68<br>5,68 |

Analog werden folgende Verbindungen erhalten:
6-(4-(2-(2,3,5,6-Tetramethylbenzolsulfonylamino)ethyl)phenyl)-6-(3-pyridyl)hex-5-ensäure
Schmelzpunkt: 135-136 °C

| $C_{29}H_{34}N_2O_4S$ (506,67) | | | | | | |
|---|---|---|---|---|---|---|
| Ber.:<br>Gef: | C | 68,75<br>68,91 | H | 6,76<br>6,81 | N | 5,33<br>5,37 |

6-(4-(2-(2,3,4,5,6-Pentamethylbenzolsulfonylamino)ethyl)phenyl)-6-(3-pyridyl)hex-5-ensäure
Schmelzpunkt: 158-160 °C (Essigsäureethylester/Diethylether)

| $C_{30}H_{36}N_2O_4S$ (520,69) | | | | | | |
|---|---|---|---|---|---|---|
| Ber.:<br>Gef: | C | 69,20<br>69,00 | H | 6,97<br>7,14 | N | 5,38<br>5,49 |

6-(4-(2-(4-Methoxybenzolsulfonylamino)ethyl)phenyl)-6-(3-pyridyl)hex-5-ensäure Schmelzpunkt: 104-106 °C

| $C_{26}H_{28}N_2O_5S$ (480,58) | | | | | | |
|---|---|---|---|---|---|---|
| Ber.:<br>Gef: | C | 64,98<br>64,90 | H | 5,87<br>6,02 | N | 5,83<br>5,99 |

6-(4-(2-(3,4-Dimethoxybenzolsulfonylamino)ethyl)phenyl)-6-(3-pyridyl)hex-5-ensäure
Ausbeute: 18 % der Theorie,
Harz, $R_f$-Wert: 0,36 (Kieselgel: Dichlormethan/Essigsäureethylester = 6:4 + 3 % Essigsäure)

| $C_{27}H_{30}N_2O_6S$ (510,61) | | | | | | |
|---|---|---|---|---|---|---|
| Ber.:<br>Gef: | C | 63,51<br>63,21 | H | 5,92<br>5,79 | N | 5,49<br>5,33 |

6-(4-(2-(4-Trifluormethylbenzolsulfonylamino)ethyl)phenyl)-6-(3-pyridyl)hex-5-ensäure
Schmelzpunkt: 140-143 °C (Essigsäureethylester/Petrolether)

| $C_{26}H_{25}F_3N_2O_4S$ (518,56) | | | | | | |
|---|---|---|---|---|---|---|
| Ber.:<br>Gef: | C | 60,22<br>60,05 | H | 4,86<br>4,77 | N | 5,40<br>5,66 |

6-(4-(2-(5-Chlorthiophen-2-sulfonylamino)ethyl)phenyl)-6-(3-pyridyl)hex-5-ensäure
Schmelzpunkt: 113-115 °C

| $C_{23}H_{23}ClN_2O_4S_2$ (491,03) | | | |
|---|---|---|---|
| Ber.:<br>Gef: | C 56,26<br>55,96 | H 4,72<br>4,70 | N 5,70<br>5,79 |

6-(4-(2-(Phenylmethansulfonylamino)ethyl)phenyl)-6-(3-pyridyl)hex-5-ensäure
$C_{26}H_{28}N_2O_4S$ (464,58)
Harz, $R_f$-Wert: 0,64 (Kieselgel: Essigsäureethylester)

| | | | | | | |
|---|---|---|---|---|---|---|
| Ber.:<br>Gef: | C | 67,22<br>67,27 | H | 6,07<br>6,22 | N | 6,03<br>5,88 |

Beispiel 31

E- und Z-6-(4-(2-(4-Chlorbenzolsulfonylamino)ethyl)phenyl)-6-(3-pyridyl)hex-5-ensäure

a) 4-(2-(4-Chlorbenzolsulfonylamino)ethyl)phenyl-3-pyridylketon

156 g 4-(2-Acetylaminoethyl)phenyl-3-pyridylketon werden in 800 ml 6N Salzsäure 16 Stunden erhitzt. Die Lösung wird eingeengt und der Rückstand in einer Mischung aus 200 ml Wasser und 500 ml Dioxan aufgenommen. Durch Zugabe von 10N Natronlauge wird ein pH von 8-10 eingestellt. Anschließend wird eine Lösung von 126 g 4-Chlorbenzolsulfonsäurechlord in 150 ml Dioxan und 10N Natronlauge bei Raumtemperatur so zugetropft, daß ein pH von 8-10 eingehalten wird. Die Reaktionsmischung wird auf ein Gemisch von 1 kg Eis und 400 ml Toluol gegeben und der Niederschlag abgesaugt. Das ausgefallene Rohprodukt wird aus Toluol umkristallisiert.
Ausbeute: 148 g (65 % der Theorie),
Schmelzpunkt: 159-160 °C

| $C_{20}H_{17}ClN_2O_3S$ (400,91) | | | | | | |
|---|---|---|---|---|---|---|
| Ber.:<br>Gef: | C | 59,92<br>60,00 | H | 4,28<br>4,10 | N | 6,99<br>6,91 |

b) E-6-(4-(2-(4-Chlorbenzolsulfonylamino)ethyl)phenyl)-6-(3-pyridyl)hex-5-ensäure

222 g 4-Carboxybutyl-triphenylphosphoniumbromid werden in 2000 ml Tetrahydrofuran suspendiert und auf -20 °C gekühlt. Zu dieser Suspension gibt man 156 g Kalium-tert.butylat und anschließend 155 g 4-(2-(4-Chlorbenzolsulfonylamino)ethyl)phenyl-3-pyridylketon. Man rührt 1,5 Stunden, wobei man die Temperatur auf 10 °C ansteigen läßt. Die Reaktionsmischung wird auf 5000 ml Eiswasser gegeben. Die Wasserphase wird mit Essigsäureethylester gewaschen und danach durch Zugabe von Zitronensäure auf einen pH von 5 eingestellt. Der Niederschlag wird abgesaugt und aus Wasser/Ethanol umkristallisiert.
Ausbeute: 140 g (75 % der Theorie),
Schmelzpunkt: 159-160 °C

| $C_{25}H_{25}ClN_2O_4S$ (485,00) | | | | | | |
|---|---|---|---|---|---|---|
| Ber.:<br>Gef: | C | 61,91<br>61,67 | H | 5,20<br>5,06 | N | 5,78<br>5,70 |

c) Z-6-(4-(2-(4-Chlorbenzolsulfonylamino)ethyl)phenyl)-6-(3-pyridyl)hex-5-ensäure

Die Mutterlauge aus Beispiel 31b wird eingeengt und mit Essigsäureethylester extrahiert. Der organische Extrakt wird eingeengt und der Rückstand über eine Kieselgelsäule mit (Ethylenchlorid/Essigsäureethylester = 6:4 + 3 % Essigsäure) chromatographiert. Die schneller laufende Fraktion wird gesammelt, eingeengt und der Rückstand aus Essigsäureethylester/Diethylether umkristallisiert.
Ausbeute: 7,8 g (3 % der Theorie),
Schmelzpunkt: 94-95 °C

| $C_{25}H_{25}ClN_2O_4S$ (485,00) | | | | | | |
|---|---|---|---|---|---|---|
| Ber.:<br>Gef: | C | 61,91<br>61,66 | H | 5,20<br>5,23 | N | 5,78<br>5,87 |

Beispiel 32

6-(2-(4-Chlorbenzolsulfonylamino)-1,2,3,4-tetrahydronaphth-6-yl)-6-(3-pyridyl)hex-5-ensäure

a) 2-Amino-6-brom-1,2,3,4-tetrahydronaphthalin-hydrochlorid

55,8 g Titantetrachlorid werden bei 0 °C vorsichtig zu 700 ml Ethylenglykoldimethylether getropft. Danach gibt man portionsweise 22,3 g Natriumborhydrid zu und anschließend eine Lösung von 33,5 g 6-Brom-2-oximino-1,2,3,4-tetrahydronaphthalin in 250 ml Ethylenglykoldimethylether. Man rührt 4 Stunden bei Raumtemperatur, gibt die Reaktionsmischung auf Eis, stellt die Mischung durch Zugabe von konz. Ammoniak alkalisch und filtriert über Kieselgur ab. Das Filtrat wird mit Methylenchlorid extrahiert. Der Extrakt wird eingeengt, der Rückstand mit Ether versetzt und durch Zugabe von isopropanolischer Salzsäure das Hydrochlorid gefällt.
Ausbeute: 20,2 g (55 % der Theorie),
Schmelzpunkt: 237 °C

| $C_{10}H_{12}BrN$ x HCl (262,5) | | | | | | |
|---|---|---|---|---|---|---|
| Ber.:<br>Gef.: | C | 45,74<br>45,90 | H | 4,99<br>5,22 | N | 5,33<br>5,24 |

b) 2-tert.Butoxycarbonylamino-6-brom-1,2,3,4-tetrahydronaphthalin

5,25 g 2-Amino-6-brom-1,2,3,4-tetrahydronaphthalin werden in 75 ml Dioxan/Wasser (2:1) gelöst. Bei 0 °C werden 44 ml 1N Natronlauge und anschließend 4,8 g Di-tert.butyldicarbonat zugegeben. Man rührt 12 Stunden bei Raumtemperatur, engt danach die Reaktionsmischung ein, versetzt mit Wasser und extrahiert mit Essigsäureethylester. Der Extrakt wird eingeengt und der Rückstand aus Petrolether umkristallisiert.
Ausbeute: 5,2 g (80 % der Theorie),
Schmelzpunkt: 111 °C

| $C_{15}H_{20}BrNO_2$ (326,23) | | | | | | |
|---|---|---|---|---|---|---|
| Ber.:<br>Gef.: | C | 55,23<br>55,08 | H | 6,18<br>6,29 | N | 4,29<br>4,51 |

c) 2-tert.Butoxycarbonylamino-6-brom-1,2,3,4-tetrahydronaphth-6-yl-3-pyridylmethanol

3,25 g 2-tert.Butoxycarbonylamino-6-brom-1,2,3,4-tetrahydronaphthalin werden in 50 ml absolutem Tetrahydrofuran gelöst und auf -70 °C gekühlt. Dazu tropft man 8,8 ml einer Lösung von n-Butyllithium in Hexan (2,5 M) und rührt weitere 1,5 Stunden bei -50 °C. Anschließend werden bei -70 °C 1,1 g Pyridin-3-aldehyd zugetropft und es wird eine weitere Stunde gerührt. Die Reaktionsmischung wird auf Eis gegeben und mit Essigsäureethylester extrahiert. Die organische Phase wird mit Wasser gewaschen, getrocknet, eingeengt und der Rückstand aus Cyclohexan/Essigsäureethylester umkristallisiert.
Ausbeute: 1,85 g (52 % der Theorie),
Schmelzpunkt: 135 °C

| $C_{21}H_{26}N_2O_3$ (354,45) | | | | | | |
|---|---|---|---|---|---|---|
| Ber.:<br>Gef.: | C | 71,16<br>70,96 | H | 7,39<br>7,46 | N | 7,90<br>7,86 |

d) 2-(4-Chlorbenzolsulfonylamino)-1,2,3,4-tetrahydronaphth-6-yl-3-pyridylketon

1,75 g 2-tert.Butoxycarbonylamino-1,2,3,4-tetrahydronaphth-6-yl-3-pyridylmethanol werden in 30 ml Chloroform mit 17,5 g Mangandioxid eine Stunde bei Raumtemperatur gerührt. Die Suspension wird filtriert, das Filtrat eingeengt, der Rückstand in 10 ml 2N Salzsäure eine Stunde bei 40-50 °C gerührt. Anschließend wird durch Zugabe von konz. Ammoniak alkalisch gestellt und die wässrige Phase mit Essigsäureethylester extrahiert. Der Extrakt wird mit Wasser gewaschen, eingeengt und der Rückstand in 20 ml Methylenchlorid gelöst. Bei 0 °C gibt man zu dieser Lösung 0,86 g 4-Chlorbenzolsulfonsäurechlorid und anschließend 1 g Triethylamin und rührt 2 Stunden bei Raumtemperatur. Die Reaktionsmischung wird auf Eis gegeben und mit Methylenchlorid extrahiert. Der Extrakt wird mit Wasser gewaschen, getrocknet, eingeengt und der Rückstand aus Essigsäureethylester/Petrolether umkristallisiert.
Ausbeute: 1,2 g (57 % der Theorie),
Schmelzpunkt: 170-172 °C

| $C_{22}H_{19}ClN_2O_3S$ (426,92) | | | | | | |
|---|---|---|---|---|---|---|
| Ber.:<br>Gef.: | C | 61,89<br>61,63 | H | 4,49<br>4,62 | N | 6,56<br>6,39 |

e) 6-(2-(4-Chlorbenzolsulfonylamino)-1,2,3,4-tetrahydronaphth-6-yl)-6-(3-pyridyl)hex-5-ensäure

Hergestellt analog Beispiel 31b aus 2-(4-Chlorbenzolsulfonylamino)-1,2,3,4-tetrahydronaphth-6-yl-3-pyridylketon und 4-Carboxybutyl-triphenylphosphoniumbromid.
Ausbeute: 63 % der Theorie,
Schmelzpunkt: 172 °C

| $C_{27}H_{27}ClN_2O_4S$ | | | | | | |
|---|---|---|---|---|---|---|
| Ber.:<br>Gef.: | C | 63,46<br>63,42 | H | 5,33<br>5,41 | N | 5,48<br>5,43 |

Beispiel I

Tabletten mit 100 mg 6-(4-(2-(4-Chlorbenzolsulfonylamino)ethyl)phenyl)-6-(3-pyridyl)hex-5-ensäure

Zusammensetzung:

| 1 Tablette enthält: | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Massen (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 9 mm, biplan mit beidseitiger Facette und einseitiger Teilkerbe. |

Beispiel II

Hartgelatine-Kapseln mit 150 mg 6-(4-(2-(4-Chlorbenzolsulfonylamino)ethyl)phenyl)-6-(3-pyridyl)hex-5-ensäure

| 1 Kapsel enthält: | |
|---|---|
| Wirkstoff | 150,0 mg |
| Maisstärke getr. ca. | 180,0 mg |
| Milchzucker pulv. ca. | 87,0 mg |
| Magnesiumstearat | 3,0 mg |
| ca. | 320,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

Beispiel III

Suppositorien mit 150 mg 6-(4-(2-(4-Chlorbenzolsulfonylamino)ethyl)phenyl)-6-(3-pyridyl)hex-5-ensäure

| 1 Zäpfchen enthält: | |
|---|---|
| Wirkstoff | 150,0 mg |
| Polyäthylenglykol (M.G. 1500) | 550,0 mg |
| Polyäthylenglykol (M.G. 6000) | 460,0 mg |
| Polyoxyäthylensorbitanmonostearat | 840,0 mg |
| | 2 000,0 mg |

Herstellung:

Nach dem Aufschmelzen der Suppositorienmassen wird der Wirkstoff darin homogen verteilt und die Schmelze in vorgekühlte Formen gegossen.

Beispiel IV

Suspensionen mit 50 mg 6-(4-(2-(4-Chlorbenzolsulfonylamino)ethyl)phenyl)-6-(3-pyridyl)hex-5-ensäure

| 100 ml Suspension enthalten: | |
|---|---|
| Wirkstoff | 1,0 g |
| Carboxymethylcellulose-Na-Salz | 0,2 g |
| p-Hydroxybenzoesäuremethylester | 0,05 g |
| p-Hydroxybenzoesäurepropylester | 0,01 g |
| Glycerin | 5,0 g |
| Sorbitlösung 70%ig | 50,0 g |
| Aroma | 0,3 g |
| Wasser dest. ad | 100 ml |

Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe der Sorbitlösung und des Aromas wird die Suspension zur Entlüftung unter Rühren evakuiert.

5 ml Suspension enthalten 50 mg Wirkstoff.

Beispiel V

Tabletten mit 150 mg 6-(4-(2-(4-Chlorbenzolsulfonylamino)ethyl)phenyl)-6-(3-pyridyl)hex-5-ensäure

Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloidale Kieselsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen. Das bei 45 ° C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

Beispiel VI

Filmtabletten mit 75 mg 6-(4-(2-(4-Chlorbenzolsulfonylamino)ethyl)phenyl)-6-(3-pyridyl)hex-5-ensäure

| 1 Tablettenkern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Tablettenkerne werden mit einem Film überzogen, der im wesentlichen aus Hydroxypropylmethylcellulose besteht. Die fertigen Filmtabletten werden mit Bienenwachs geglänzt.

| | |
|---|---|
| Filmtablettengewicht: | 245 mg |

Selbstverständlich können alle übrigen Verbindungen der allgemeinen Formel I als Wirkstoffe in den vorstehenden galenischen Zubereitungen eingesetzt werden.

Beispiel VII

Filmtabletten, enthaltend 75 mg 6-(4-(2-(4-Chlorbenzolsulfonylamino)ethyl)phenyl)-6-(3-pyridyl)hex-5-ensäure (Substanz B) + 75 mg PDE-Hemmer

Eine Pulvermischung aus

| | |
|---|---|
| Dipyridamol | 25 % |
| Substanz B | 25 % |
| Fumarsäure | 15 % |
| Cellulose | 20 % |
| Maisstärke | 8 % |
| Polyvinylpyrrolidon | 6 % |

wird in einem Mischgerät mit Wasser befeuchtet und durch ein Sieb mit der Maschenweite 1.5 mm granuliert. Nach Trocknung und erneuter Siebung mischt man 1 % Magnesiumstearat zu und stellt 10 mm bikonvexe Tabletten von 300 mg her. Diese Tabletten werden solange mit Hydroxypropylmethylcelluloselack besprüht bis sie 312 mg wiegen.

Beispiel VIII

Hartgelatinekapseln, enthaltend 200 mg 6-(4-(2-(4-Chlorbenzolsulfonylamino)ethyl)phenyl)-6-(3-pyridyl)hex-5-ensäure (Substanz B) + 50 mg PDE-Hemmer

10 kg Dipyridamol, 20 kg Fumarsäure, 11,5 kg Polyvinylpyrrolidon, 40 kg Substanz B, 1,5 kg Siliciumdioxid und 0,8 kg Magnesiumstearat mischt man 15 Minuten in einem Kubusmischer. Diese Mischung gibt man über einen Walzenkompaktor, dem ein Trockengranuliergerät mit Siebeinrichtung nachgeschaltet ist. Verwendung findet die Fraktion 0.25-1,0 mm. Die Kapselfüllmaschine wird so eingestellt, daß jede Kapsel der Größe 0 die 50 mg PDE-Hemmer und 200 mg (Substanz B) entsprechende Menge Granulat enthält.

Beispiel IX

Hartgelatinekapseln enthaltend 100 mg 6-(4-(2-(4-Chlorbenzolsulfonylamino)ethyl)phenyl)-6-(3-pyridyl)hex-5-ensäure (Substanz B) + 250 mg PDE-Hemmer

a) Granulat

125 kg Mopidamol, 50 kg Fumarsäure, 13,5 kg Milchzucker werden gemischt und mit einer Lösung aus Wasser/Polyäthylenglykol 6000 befeuchtet. Nach Granulierung durch ein Sieb mit der Maschenweite 1,0 mm und Trocknung bei 45 °C mischt man 1,4 kg Stearinsäure zu.

b) Dragée

100 kg Substanz B, 7,5 kg Hydroxypropylmethylcellulose, 2,5 kg Siliciumdioxid und 15 kg Carboxymethylcellulose werden mit Äthanol befeuchtet und durch ein Sieb mit der Maschenweite 1,5 mm granuliert. Nach Trocknung mischt man 1 kg Magnesiumstearat zu und verpreßt das Granulat zu 126 mg schweren bikonvexen Tabletten mit einem Durchmesser von 5,5 mm.

Diese Kerne überzieht man in mehreren Schritten mit einer Dragiersuspension, bestehend aus 5,6 kg Saccharose, 0,5 kg Gummi-arabicum und 3,8 kg Talcum, bis die Tabletten ein Gewicht von 135 mg haben.

c) Abfüllung

Auf einer Spezial-Kapselmaschine füllt man in eine Hartgelatine-Kapsel der Größe 0 long die 250 mg PDE-Hemmer entsprechende Menge Granulat ein und legt das 100 mg Substanz B enthaltende Dragée obenauf.

Beispiel X

Suspension, enthaltend 10 mg 6-(4-(2-(4-Chlorbenzolsulfonylamino)ethyl)phenyl)-6-(3-pyridyl)hex-5-ensäure (Substanz B) + 100 mg Dipyridamol pro 5 g.

Die Suspension hat folgende Zusammensetzung:

| | |
|---|---|
| (1) Dipyridamol | 2,0 % |
| (2) Substanz B | 0,2 % |
| (3) Sorbit | 20,8 % |
| (4) Cellulose | 7,5 % |
| (5) Natriumcarboxymethylcellulose | 2,5 % |
| (6) Geschmackskorrigentien/Konservierungsstoffe | 1,8 % |
| (7) Wasser | 65,2 % |

In heißes Wasser wird unter hoher Scherung (3) - (6) eingerührt. Nach Abkühlung werden in die viskose Suspension (1), (2) und (7) eingearbeitet.

Beispiel XI

Depot-Form, enthaltend 50 mg 6-(4-(2-(4-Chlorbenzolsulfonylamino)ethyl)phenyl)-6-(3-pyridyl)hex-5-ensäure (Substanz B) + 200 mg Dipyridamol

a) Pellet I

Eine Mischung aus

| | |
|---|---|
| Substanz B | 50,0 kg |
| Lysin | 12,5 kg |
| Hydroxypropylcellulose, hochpolymer | 52,5 kg |
| Triacetin | 4,0 kg |
| Äthylcellulose | 2,5 kg |
| Magnesiumstearat | 3,5 kg |

wird auf einem Spezialextruder mit Äthanol geknetet und in Form von Spaghetti (Durchmesser 1 mm) extrudiert, die in einem Sphäronizer zu Pellets gerundet werden. Danach trocknet man sie gründlich.

b) Pellet II

300 kg ausgemischte Weinsäure-Starterpellets werden in einem Spezialkessel mit einer Suspension bestehend aus Isopropanol, Dipyridamol und Polyvinylpyrrolidon solange besprüht, bis die entstehenden

Wirkstoffpellets ca. 45 % Dipyridamol enthalten.

Diese Pellets besprüht man mit einem Lack, der aus Methacrylsäure/Methylmethacrylat-Copolymer (Handelsnahme Eudragit S) und Hydroxypropylmethylcellulosephthalat (Handelsname HP 55) im Gewichtsverhältnis 85:15 bis 50:50 besteht. Die organischen Lacklösungen enthalten noch Weichmacher und Talkum. Es werden zwei Pelletkomponenten mit 5 und 7 % Hüllenmittel und unterschiedlichem Verhältnis der Lackkomponenten in den genannten Grenzen gesprüht. Die beiden Komponenten werden so gemischt, daß sie nachfolgende in vitro-Freigabe ergeben:

Bedingungen (entsprechend USPXXI, Basket-Methode, 100 Umdrehungen/Min.,

1. Stunde künstlicher Magensaft, pH 1,2, 2. bis 6. Stunde künstlicher Darmsaft (Phosphatpuffer), pH 5.5):

Wirkstoff-Freigabe pro Stunde:

1. Stunde ca. 30 %
2. Stunde ca. 25 %
3. Stunde ca. 18 %
4. Stunde ca. 12 %

nach der 6. Stunde mehr als 90 % Dipyridamol-Freigabe.

c) Abfüllung

Entsprechend dem Wirkstoffgehalt der Pelletkomponenten I und II und der gewünschten Dosierung werden die Pellets miteinander vermischt und auf einer Kapselmaschine in Kapseln der Größe 0 long abgefüllt.

Beispiel XII

Ampullen, enthaltend 5 mg 6-(4-(2-(4-Chlorbenzolsulfonylamino)ethyl)phenyl)-6-(3-pyridyl)hex-5-ensäure (Substanz B) + 10 mg Dipyridamol per 5 ml

Zusammensetzung:

| | |
|---|---|
| (1) Dipyridamol | 10 mg |
| (2) Substanz B | 5 mg |
| (3) Propylenglykol | 50 mg |
| (4) Polyethylenglykol | 5 mg |
| (5) Ethanol | 10 mg |
| (6) Wasser für Injektionszwecke ad | 5 ml |
| (7) 1N HCl ad | pH 3 |

Die Wirkstoffe werden unter Erwärmung in der Lösung aus (3) - (7) gelöst. Nach pH-Kontrolle und Sterilfiltration füllt man in geeignete Ampullen und sterilisiert.

**Patentansprüche**

**1.** Arylsulfonamide der Formel

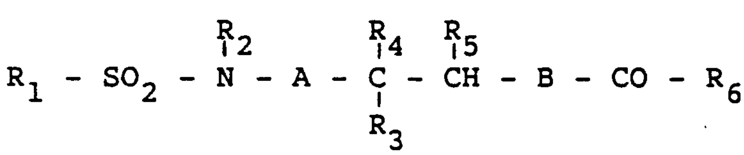

,(I)

in der

$R_1$ eine Phenylalkyl-, Trialkylphenyl-, Tetramethylphenyl-oder Pentamethylphenylgruppe, eine gegebenenfalls durch ein Halogenatom oder eine Alkylgruppe substituierte Thienylgruppe oder eine Phenylgruppe, die durch eine Nitrogruppe monosubstituiert oder durch ein Halogenatom, eine Alkyl-, Trifluormethyl- oder Alkoxygruppe mono- oder disubstituiert sein kann, wobei die Substituenten gleich oder

verschieden sein können,
$R_2$, $R_4$ und $R_5$ die gleich oder verschieden sein können, jeweils ein Wasserstoffatom oder eine Alkylgruppe oder
$R_2$ ein Wasserstoffatom oder eine Alkylgruppe und $R_4$ und
$R_5$ zusammen eine Kohlenstoff-Kohlenstoff-Bindung,
$R_3$ eine gegebenenfalls durch eine Alkylgruppe substituierte Pyridylgruppe,
$R_6$ eine Hydroxy-, Alkoxy-, Amino-, Alkylamino- oder Dialkylaminogruppe,
A eine Gruppe der Formeln

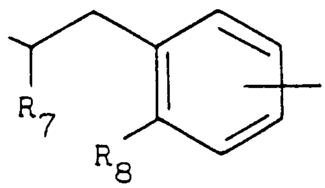 , 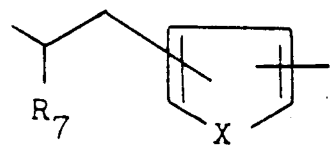 oder 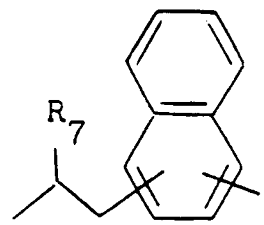 ,

in denen
$R_7$ ein Wasserstoffatom oder eine Alkylgruppe,
$R_8$ ein Wasserstoffatom oder
$R_7$ und $R_8$ zusammen eine Methylen- oder Ethylengruppe und
X eine durch eine Alkylgruppe substituierte Iminogruppe, ein Sauerstoff- oder Schwefelatom darstellen, wobei die -$CHR_7$-Gruppe mit der -$NR_2$-Gruppe verknüpft ist, und
B eine Kohlenstoff-Kohlenstoff-Bindung oder eine gegebenenfalls durch eine oder zwei Alkylgruppen substituierte geradkettige Alkylengruppe mit 1 bis 4 Kohlenstoffatomen bedeuten, wobei alle vorstehend erwähnten Alkyl- und Alkoxyteile jeweils 1 bis 3 Kohlenstoffatome enthalten können, deren Enantiomere, deren cis- und trans-Isomere, sofern $R_4$ und $R_5$ zusammen eine Kohlenstoff-Kohlenstoff-Bindung darstellen, und deren Additionssalze.

**2.** Arylsulfonamide der Formel I gemäß Anspruch 1, in der
$R_1$ eine Benzyl-, Thienyl-, Chlorthienyl-, Dichlorphenyl-, Dimethoxyphenyl-, Tetramethylphenyl- oder Pentamethylphenylgruppe oder eine gegebenenfalls durch ein Fluor- oder Chloratom, eine Nitro-, Methyl- oder Trifluormethylgruppe substituierte Phenylgruppe,
$R_2$, $R_4$ und $R_5$ jeweils ein Wasserstoffatom oder eine Methylgruppe oder
$R_2$ ein Wasserstoffatom oder eine Methylgruppe und $R_4$ und $R_5$ zusammen eine Kohlenstoff-Kohlenstoff-Bindung,
$R_3$ eine Pyridylgruppe,
$R_6$ eine Hydroxy- oder Methoxygruppe,
A eine Gruppe der Formeln

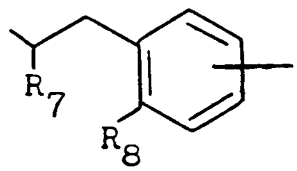 , 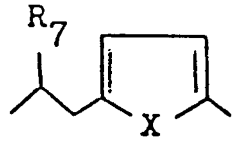 oder 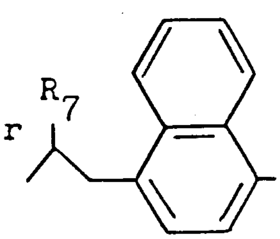 ,

in denen
$R_7$ und $R_8$ jeweils ein Wasserstoffatom oder zusammen eine Methylen- oder Ethylengruppe und
X ein Schwefelatom oder die N-Methyliminogruppe darstellen, wobei die -$CHR_7$-Gruppe mit der -$NR_2$-Gruppe verknüpft ist, und
B eine Kohlenstoff-Kohlenstoff-Bindung oder eine geradkettige Alkylengruppe mit 2 bis 4 Kohlenstoffatomen bedeuten, deren Enantiomere, deren cis- und trans-Isomeren, sofern $R_4$ und $R_5$ zusammen eine Kohlenstoff-Kohlenstoff-Bindungbilden, und deren Additionssalze.

**3.** Arylsulfonamide der Formel I gemäß Anspruch 1, in der
B wie im Anspruch 1 oder 2 definiert ist,
$R_1$ eine Tetramethylphenyl- oder Pentamethylphenylgruppe oder eine in 4-Stellung durch eine Methylgruppe, eine Trifluormethylgruppe, ein Fluor-, Chlor- oder Bromatom substituierte Phenylgruppe,
$R_2$, $R_4$ und $R_5$ jeweils ein Wasserstoffatom oder
$R_2$ ein Wasserstoffatom und $R_4$ und $R_5$ zusammen eine Kohlenstoff-Kohlenstoff-Bindung,
$R_3$ eine 3-Pyridylgruppe,
A eine Gruppe der Formel

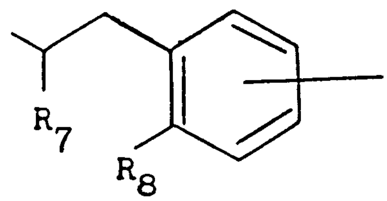

in der
$R_7$ und $R_8$ jeweils ein Wasserstoffatom oder
$R_7$ und $R_8$ zusammen eine Methylengruppe darstellen, und
$R_6$ die Hydroxygruppe bedeuten, deren Enantiomere, deren cis- und trans-Isomeren, sofern $R_4$ und $R_5$ zusammen eine Kohlenstoff-Kohlenstoff-Bindung bilden, und deren Additionssalze.

**4.** Als neue Verbindungen der Formel I gemäß Anspruch 1:
6-(2-(4-Toluolsulfonylamino)indan-5-yl)-6-(3-pyridyl)hex-5-ensäure,
6-(4-(2-(4-Chlorbenzolsulfonylamino)ethyl)phenyl)-6-(3-pyridyl)hex-5-ensäure und
6-(4-(2-(4-Trifluormethylbenzolsulfonylamino)ethyl)phenyl)-6-(3-pyridyl)hex-5-ensäure und deren Säureadditionssalze.

**5.** 6-(4-(2-(4-Chlorbenzolsulfonylamino)ethyl)phenyl)-6-(3-pyridyl)hex-5-ensäure und dessen Additionssalze.

**6.** Physiologisch verträgliche Additionssalze der Verbindung gemäß den Ansprüchen 1 bis 5 mit anorganischen oder organischen Basen.

**7.** Arzneimittel, enthaltend als Wirkstoff eine Verbindung gemäß den Ansprüchen 1 bis 5 oder dessen physiologisch verträgliches Additionssalz gemäß Anspruch 6 neben gegebenenfalls einem oder mehreren inerten Trägerstoffen und/oder Verdünnungsmitteln.

**8.** Arzneimittel gemäß Anspruch 7 geeignet zur Behandlung und zur Prophylaxe thrombo-embolischer Erkrankungen, zur Prophylaxe der Arteriosklerose und zur Metastasenprophylaxe sowie zur Behandlung der Ischämie, des Asthmas und von Allergien.

**9.** Arzneimittel gemäß Anspruch 7 geeignet zur Behandlung und zur Prophylaxe von Erkrankungen bei denen eine thromboxanvermittelte Konstriktion oder $PGE_2$ vermittelte Dilatation der Kapillaren eine Rolle spielen, zur Verminderung des Schweregrades einer Transplantatabstoßung, zur Verminderung der renalen Toxizität von Substanzen wie Cyclosporin, zur Behandlung von Nierenerkrankungen und zur Behandlung von Schockzuständen.

**10.** Arzneimittel gemäß Anspruch 7, 8 oder 9 dadurch gekennzeichnet, daß dieses zusätzlich als Wirkstoff einen PDE-Hemmer oder ein Lysemittel enthalten.

**11.** Verfahren zur Herstellung eines Arzneimittels gemäß den Ansprüchen 7 bis 10, dadurch gekennzeichnet, daß auf nicht-chemischem Wege eine Verbindung gemäß den Ansprüchen 1 bis 5 oder dessen physiologisch verträgliches Additionssalz gemäß Anspruch 6 gegebenenfalls in Kombination mit einem PDE-Hemmer oder einem Lysemittel in einen oder mehrere inerte übliche Trägerstoffe und/oder Verdünnungsmittel eingearbeitet wird.

**12.** Verfahren zur Herstellung der Verbindungen gemäß den Ansprüchen 1 bis 6, dadurch gekennzeichnet, daß

a.) eine Verbindung der Formel

$$H - \underset{R_2}{N} - A - \overset{R_4}{\underset{R_3}{C}} - \overset{R_5}{CH} - B - CO - R_6 \qquad ,(II)$$

in der
$R_2$ bis $R_6$, A und B wie in einem der Ansprüche 1 bis 5 definiert sind, mit einem Sulfonsäurederivat der Formel

$R_1$ - $SO_2X$ ,(III)

in der
$R_1$ wie in einem der Ansprüche 1 bis 5 definiert ist und
X eine nucleophile Austrittsgruppe wie ein Halogenatom oder eine Alkoxygruppe, z.B. ein Chlor- oder Bromatom, eine Methoxy- oder Äthoxygruppe, darstellt, acyliert wird oder
b.) zur Herstellung von Verbindungen der Formel I, in der $R_6$ eine Hydroxygruppe darstellt, von einer Verbindung der Formel

$$R_1 - SO_2 - \overset{R_2}{N} - A - \overset{R_4}{\underset{R_3}{C}} - \overset{R_5}{CH} - B - CO - Z \qquad ,(IV)$$

in der
$R_1$ bis $R_5$, A und B wie in einem der Ansprüche 1 bis 5 definiert sind und
Z eine hydrolytisch, thermolytisch oder hydrogenolytisch abspaltbare Schutzgruppe für eine Carboxygruppe oder ein funktionelles Derivat der Carboxygruppe darstellt, ein Schutzrest abgespalten wird oder
c.) zur Herstellung von Verbindungen der Formel I, in der $R_4$ und $R_5$ jeweils ein Wasserstoffatom darstellen, eine Verbindung der Formel

$$R_1 - SO_2 - \overset{R_2}{N} - A - \underset{R_3}{C} = CH - B - CO - R_6 \qquad ,(V)$$

in der
$R_1$ bis $R_3$, $R_6$, A und B wie in einem der
Ansprüche 1 bis 5 definiert sind, hydriert wird oder d.) zur Herstellung von Verbindungen der Formel I, in der
$R_4$ und $R_5$ zusammen eine Kohlenstoff-Kohlenstoff-Bindungdarstellen, eine Verbindung der Formel

$$R_1 - SO_2 - \overset{R_2}{N} - A - CO - R_3 \qquad ,(VI)$$

in der

$R_1$ bis $R_3$ und A wie in einem der Ansprüche 1 bis 5 definiert sind, mit einer Verbindung der Formel

$$\begin{array}{l} \quad\;\; R_5{}' \\ \quad\;\; | \\ W - CH - B - CO - R_6 \end{array} \qquad ,(VII)$$

in der
B und $R_6$ wie in einem der Ansprüche 1 bis 5 definiert sind,
$R_5{}'$ ein Wasserstoffatom oder eine Alkylgruppe mit 1 bis 3 Kohlenstoffatomen und
W einen Triphenylphosphoniumhalogenid-, einem Dialkylphosphonsäure- oder ein Magnesiumhalogenidrest bedeuten, umgesetzt und gegebenenfalls anschließend dehydratisiert wird und
gewünschtenfalls anschließend eine so erhaltene Verbindung der Formel I, in der $R_2$ ein Wasserstoffatom darstellt, mittels Alkylierung in eine entsprechende Verbindung der Formel I, in der $R_2$ eine Alkylgruppe darstellt, übergeführt wird oder
eine so erhaltene Verbindung der Formel I, in der $R_6$ eine Hydroxygruppe darstellt, mittels Veresterung oder Amidierung in eine entsprechende Verbindung der Formel I, in der $R_6$ eine Alkoxy-, Amino-, Alkylamino- oder Dialkylaminogruppe darstellt, übergeführt wird oder
eine so erhaltene Verbindung der Formel I, in der $R_4$ und $R_5$ zusammen eine Kohlenstoff-Kohlenstoff-Bindung darstellen, in ihre cis- und trans-Isomere aufgetrennt wird oder
eine so erhaltene Verbindung der Formel I in ihre Enantiomeren aufgetrennt wird oder
eine so erhaltene Verbindung der Formel I in ihre Additionssalze, insbesondere in ihre physiologisch verträglichen Additionssalze mit anorganischen oder organischen Basen übergeführt wird.

## Claims

**1.** Arylsulphonamides of formula

$$\begin{array}{ccccccccccccccccc} & & & R_2 & & & & R_4 & & R_5 & & & & & & \\ & & & | & & & & | & & | & & & & & & \\ R_1 & - & SO_2 & - N & - & A & - & C & - & CH & - & B & - & CO & - & R_6 \\ & & & & & & & | & & & & & & & & \\ & & & & & & & R_3 & & & & & & & & \end{array} \qquad (I)$$

wherein
$R_1$ represents a phenylalkyl, trialkylphenyl, tetramethylphenyl or pentamethylphenyl group, a thienyl group optionally substituted by a halogen atom or an alkyl group, or a phenyl group which may be mono-substituted by a nitro group or mono- or disubstituted by a halogen atom or by an alkyl, trifluoromethyl or alkoxy group, the substituents being identical or different,
$R_2$, $R_4$ and $R_5$, which may be identical or different, each represents a hydrogen atom or an alkyl group or
$R_2$ represents a hydrogen atom or an alkyl group and $R_4$ and $R_5$ together represent a carbon-carbon bond,
$R_3$ represents a pyridyl group optionally substituted by an alkyl group,
$R_6$ represents a hydroxy, alkoxy, amino, alkylamino or dialkylamino group,
A represents a group of formula

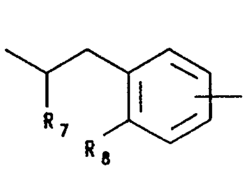
or
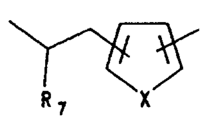
or
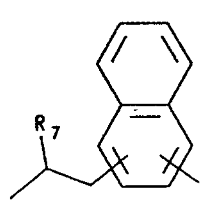

wherein

$R_7$ represents a hydrogen atom or an alkyl group,
$R_8$ represents a hydrogen atom or
$R_7$ and $R_8$ together represent a methylene or ethylene group and
X represents an alkyl-substituted imino group or an oxygen or sulphur atom, the -$CHR_7$- group being attached to the -$NR_2$- group, and
B represents a carbon-carbon bond or a straight-chained $C_{1-4}$ alkylene group optionally substituted by one or two alkyl groups, whilst all the alkyl and alkoxy moieties mentioned hereinbefore may each contain 1 to 3 carbon atoms,
the enantiomers thereof, the cis- and trans- isomers thereof where $R_4$ and $R_5$ together represent a carbon-carbon bond, and the addition salts thereof.

**2.** Arylsulphonamides of formula I according to claim 1 wherein
$R_1$ represents a benzyl, thienyl, chlorothienyl, dichlorophenyl, dimethoxyphenyl, tetramethylphenyl or pentamethylphenyl group or a phenyl group optionally substituted by a fluorine or chlorine atom or by a nitro, methyl or trifluoromethyl group,
$R_2$, $R_4$ and $R_5$ each represent a hydrogen atom or a methyl group or
$R_2$ represents a hydrogen atom or a methyl group and $R_4$ and $R_5$ together represent a carbon-carbon bond,
$R_3$ represents a pyridyl group,
$R_6$ represents a hydroxy or methoxy group,
A represents a group of formula

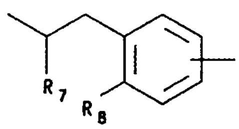
or
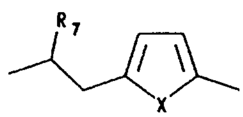
or
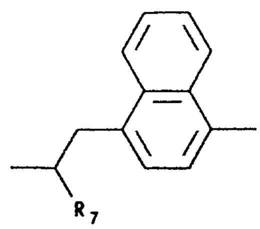

wherein
$R_7$ and $R_8$ each represent a hydrogen atom or together represent a methylene or ethylene group and
X represents a sulphur atom or an N-methylimino group, the -$CHR_7$- group being linked to the -$NR_2$- group, and
B represents a carbon-carbon bond or a straight-chained $C_{2-4}$ alkylene group,
the enantiomers, the cis and trans isomers where $R_4$ and $R_5$ together form a carbon-carbon bond, and the addition salts thereof.

**3.** Arylsulphonamides of formula I according to claim 1, wherein
B is as defined in claim 1 or 2,
$R_1$ represents a tetramethylphenyl or pentamethylphenyl group or a phenyl group substituted in the 4-position by a methyl or trifluoromethyl group or by a fluorine, chlorine or bromine atom,
$R_2$, $R_4$ and $R_5$ each represents a hydrogen atom or
$R_2$ represents a hydrogen atom and $R_4$ and $R_5$ together represent a carbon-carbon bond,
$R_3$ represents a 3-pyridyl group,
A represents a group of the formula

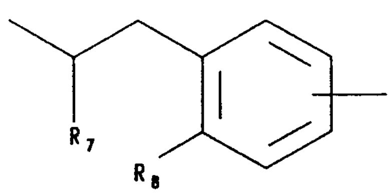

wherein

$R_7$ and $R_8$ each represents a hydrogen atom or
$R_7$ and $R_8$ together represent a methylene group, and
$R_6$ represents a hydroxy group,
the enantiomers, the cis and trans isomers thereof where $R_4$ and $R_5$ together form a carbon-carbon bond, and the addition salts thereof.

**4.** As new compounds of formula I according to claim 1:
6-(2-(4-toluenesulphonylamino)indan-5-yl)-6-(3-pyridyl)-hex-5-enoic acid,
6-(4-(2-(4-chlorobenzenesulphonylamino)ethyl)phenyl)-6-(3-pyridyl)hex-5-enoic acid and
6-(4-(2-(4-trifluoromethylbenzenesulphonylamino)-ethyl)phenyl)-6-(3-pyridyl)hex-5-enoic acid
and the acid addition salts thereof.

**5.** 6-(4-(2-(4-Chlorobenzenesulphonylamino)ethyl)-phenyl)-6-(3-pyridyl)hex-5-enoic acid and the acid addition salts thereof.

**6.** Physiologically acceptable addition salts of the compounds according to claims 1 to 5 with inorganic or organic bases.

**7.** Pharmaceutical composition containing as active substance a compound according to claims 1 to 5 or a physiologically acceptable addition salt thereof according to claim 6, optionally together with one or more inert carriers and/or diluents.

**8.** Pharmaceutical composition according to claim 7 suitable for the treatment and prevention of thromboembolic disorders, for the prevention of arteriosclerosis and metastasis and for treating ischaemia, asthma and allergies.

**9.** Pharmaceutical composition according to claim 7 suitable for the treatment and prevention of diseases in which thromboxane-mediated constriction or $PGE_2$-mediated dilation of the capillaries are involved, in order to reduce the severity of transplant rejection, to reduce renal toxicity of substances such as cyclosporin, for treating kidney diseases and for treating states of shock.

**10.** Pharmaceutical composition according to claim 7, 8 or 9, characterised in that it additionally contains as active substance a PDE inhibitor or a lysing agent.

**11.** Process for preparing a pharmaceutical composition as claimed in claims 7 to 10, characterised in that a compound according to claims 1 to 5 or a physiologically acceptable addition salt thereof according to claim 6, optionally in conjunction with a PDE inhibitor or a lysing agent, is incorporated in one or more inert conventional carriers and/or diluents by a non-chemical method.

**12.** Process for preparing compounds according to claims 1 to 6, characterised in that
a) a compound of formula

```
                R4   R5
                |    |
H - N - A - C - CH - B - CO - R6          (II)
    |       |
    R2      R3
```

(wherein $R_2$ to $R_6$, A and B are as defined in one of claims 1 to 5) is acylated with a sulphonic acid derivative of formula

$R_1$ - $SO_2X$ (III)

wherein
$R_1$ is as defined in one of claims 1 to 5 and

X represents a nucleophilic leaving group such as a halogen atom or an alkoxy group, e.g. a chlorine or bromine atom or a methoxy or ethoxy group, or

b) in order to prepare compounds of formula I wherein $R_6$ represents a hydroxy group, a protecting group is split off from a compound of formula

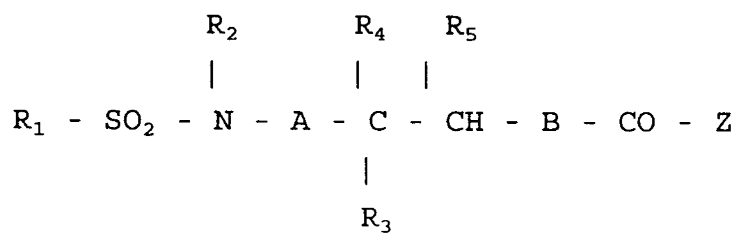

(IV)

wherein

$R_1$ to $R_5$, A and B are as defined in one of claims 1 to 5 and

Z represents a hydrolytically, thermolytically or hydrogenolytically removable protecting group for a carboxy group or a functional derivative of the carboxy group, or

c) in order to prepare compounds of formula I wherein $R_4$ and $R_5$ each represent a hydrogen atom, a compound of formula

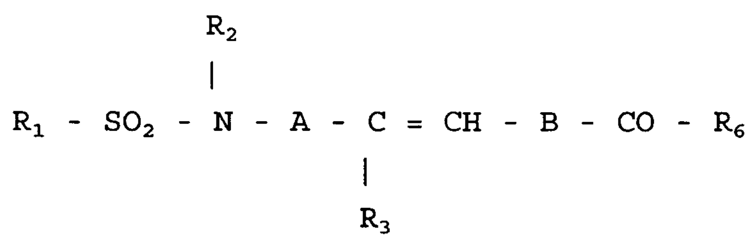

(V)

(wherein $R_1$ to $R_3$, $R_6$, A and B are as defined in one of claims 1 to 5) is hydrogenated or

d) in order to prepare compounds of formula I wherein $R_4$ and $R_5$ together represent a carbon-carbon bond, a compound of formula

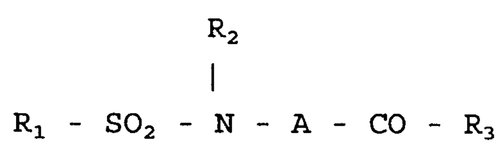

(VI)

(wherein $R_1$ to $R_3$ and A are as defined in one of claims 1 to 5) is reacted with a compound of formula

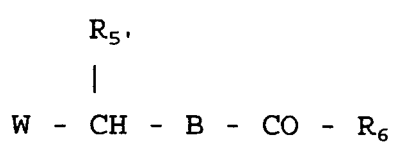

(VII)

(wherein B and $R_6$ are as defined in one of claims 1 to 5,

$R_5'$ represents a hydrogen atom or a $C_{1-3}$ alkyl group and

W represents a triphenylphosphonium halide, a dialkylphosphonic acid or magnesium halide group) and is optionally subsequently dehydrated and

subsequently, if desired, a compound of formula I thus obtained wherein $R_2$ represents a hydrogen atom is converted by alkylation into a corresponding compound of formula I wherein $R_2$ represents an alkyl group, or

a compound of formula I thus obtained wherein $R_6$ represents a hydroxy group may be converted by

esterification or amidation into a corresponding compound of formula I wherein $R_6$ represents an alkoxy, amino, alkylamino or dialkylamino group, or
a compound of formula I thus obtained wherein $R_4$ and $R_5$ together represent a carbon-carbon bond is resolved into the cis- and trans-isomers thereof or
a compound of formula I thus obtained is resolved into the enantiomers thereof or
a compound of formula I thus obtained is converted into the addition salts thereof, more particularly the physiologically acceptable addition salts thereof with organic or inorganic bases.

## Revendications

1. Arylsulfonamides de formule

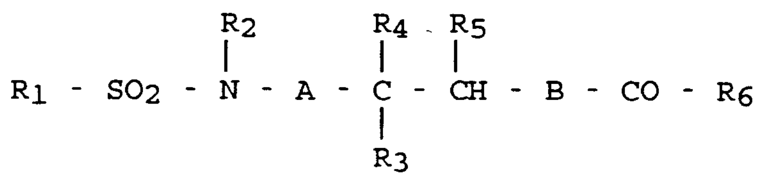 (I)

dans laquelle
$R_1$ représente un groupe phénylalkyle, trialkylphényle, tétraméthylphényle ou pentaméthylphényle, un groupe thiényle éventuellement substitué par un atome d'halogène ou un groupe alkyle ou un groupe phényle qui peut être monosubstitué par un groupe nitro ou qui peut être mono- ou disubstitué par un atome d'halogène, un groupe alkyle, trifluorométhyle ou alcoxy, les substituants pouvant être identiques ou différents,
$R_2$, $R_4$ et $R_5$, qui peuvent être identiques ou différents, représentent chacun un atome d'hydrogène ou un groupe alkyle ou
$R_2$ représente un atome d'hydrogène ou un groupe alkyle et $R_4$ et $R_5$ représentent ensemble une liaison carbone-carbone,
$R_3$ représente un groupe pyridyle éventuellement substitué par un groupe alkyle,
$R_6$ représente un groupe hydroxyle, alcoxy, amino, alkylamino ou dialkylamino,
A représente un groupe selon les formules

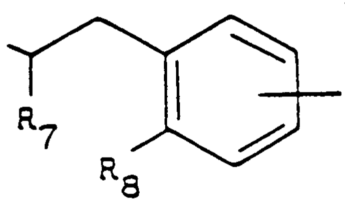 , 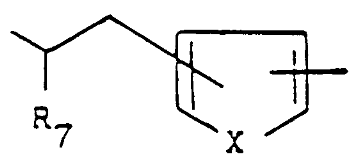 ou 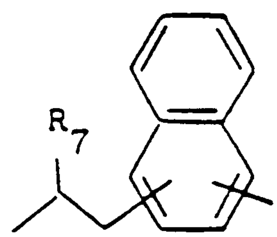 ,

dans lesquelles
$R_7$ représente un atome d'hydrogène ou un groupe alkyle,
$R_8$ représente un atome d'hydrogène ou
$R_7$ et $R_8$ représentent ensemble un groupe méthylène ou éthylène et
X représente un groupe imino substitué par un groupe alkyle, un atome d'oxygène ou de soufre, le groupe $-CHR_7-$ étant lié au groupe $-NR_2-$, et
B représente une liaison carbone-carbone ou un groupe alkylène linéaire de 1 à 4 atomes de carbone éventuellement substitué par un ou deux groupes alkyle, toutes les parties alkyle et alcoxy citées précédemment pouvant contenir chacune 1 à 3 atomes de carbone, leurs énantiomères, leurs isomères cis et trans, dans la mesure où $R_4$ et $R_5$ représentent ensemble une liaison carbone-carbone, et leurs sels d'addition.

2. Arylsulfonamides de formule I selon la revendication 1, dans laquelle
$R_1$ représente un groupe benzyle, thiényle, chlorothiényle, dichlorophényle, diméthoxyphényle, tétraméthylphényle ou pentaméthylphényle ou un groupe phényle éventuellement substitué par un atome de fluor ou de chlore, un groupe nitro, méthyle ou trifluorométhyle,

$R_2$, $R_4$ et $R_5$ représentent chacun un atome d'hydrogène ou un groupe méthyle ou
$R_2$ représente un atome d'hydrogène ou un groupe méthyle et
$R_4$ et $R_5$ représentent ensemble une liaison carbone-carbone,
$R_3$ représente un groupe pyridyle,
$R_6$ représente un groupe hydroxyle ou méthoxy,
A représente un groupe selon les formules

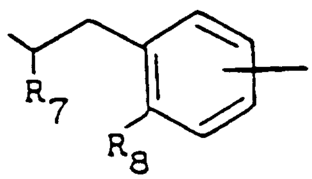 , 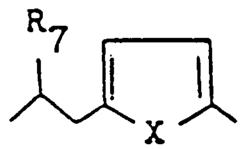 ou 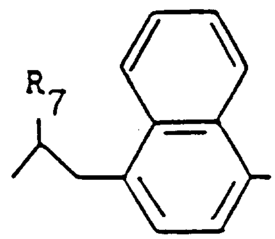 ,

dans lesquelles
$R_7$ et $R_8$ représentent chacun un atome d'hydrogène ou ensemble un groupe méthylène ou éthylène et :
X représente un atome de soufre ou le groupe N-méthylimino, le groupe $-CHR_7-$ étant lié au groupe $-NR_2-$, et
B représente une liaison carbone-carbone ou un groupe alkylène linéaire de 2 à 4 atomes de carbone, leurs énantiomères, leurs isomères cis et trans, dans la mesure où
$R_4$ et $R_5$ représentent ensemble une liaison carbone-carbone, et leurs sels d'addition.

**3.** Arylsulfonamides de formule I selon la revendication 1, dans laquelle
B est défini comme dans la revendication 1 ou 2,
$R_1$ représente un groupe tétraméthylphényle ou pentaméthylphényle ou un groupe phényle substitué en position 4 par un groupe méthyle, un groupe trifluorométhyle, un atome de fluor, de chlore ou de brome,
$R_2$, $R_4$ et $R_5$ représentent chacun un atome d'hydrogène ou
$R_2$ représente un atome d'hydrogène et $R_4$ et $R_5$ représentent ensemble une liaison carbone-carbone,
$R_3$ représente un groupe 3-pyridyle,
A représente un groupe de formule

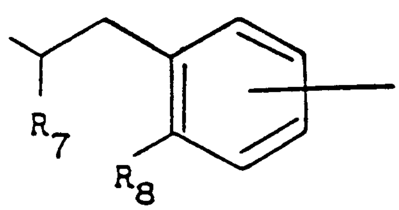

dans laquelle
$R_7$ et $R_8$ représentent chacun un atome d'hydrogène ou
$R_7$ et $R_8$ représentent ensemble un groupe méthylène et
$R_6$ représente le groupe hydroxyle, leurs énantiomères, leurs isomères cis et trans, dans la mesure où
$R_4$ et $R_5$ représentent ensemble une liaison carbone-carbone, et leurs sels d'addition.

**4.** En tant que nouveaux composés de formule I selon la revendication 1:
acide 6-(2-(4-toluènesulfonylamino)indan-5-yl)-6-(3-pyridyl)hex-5-énoïque,
acide 6-(4-(2-(4-chlorobenzènesulfonylamino)éthyl)phényl)-6-(3-pyridyl)hex-5-énoïque et
acide 6-(4-(2-(4-trifiuorométhylbenzènesulfonylamino)éthyl)-phényl)-6-(3-pyridyl)hex-5-énoïque et leurs sels d'addition.

**5.** Acide 6-(4-(2-(4-chlorobenzènesulfonylamino)éthyl)-phényl)-6-(3-pyridyl)hex-5-énoïque et ses sels d'addition.

**6.** Sels d'addition physiologiquement acceptables du composé selon les revendications 1 à 5 avec des bases inorganiques ou organiques.

**7.** Médicament contenant comme principe actif un composé selon les revendications 1 à 5 ou son sel d'addition physiologiquement acceptable selon la revendication 6 et éventuellement un ou plusieurs supports et/ou diluants inertes.

**8.** Médicament selon la revendication 7 qui convient pour le traitement et pour la prophylaxie des maladies thrombo-emboliques, pour la prophylaxie de l'artériosclérose et pour la prophylaxie des métastases ainsi que pour le traitement de l'ischémie, de l'asthme et des allergies.

**9.** Médicament selon la revendication 7 qui convient pour le traitement et pour la prophylaxie des maladies dans lesquelles une constriction des capillaires provoquée par les thronboxanes ou une dilatation des capillaires provoquée par $PGE_2$ joue un rôle, pour réduire le degré de gravité d'un rejet de greffe, pour réduire la toxicité rénale de substances comme la cyclosporine, pour le traitement des maladies rénales et pour le traitement des états de choc.

**10.** Médicament selon la revendication 7, 8 ou 9 caractérisé en ce qu'il contient en outre comme principe actif un inhibiteur de PDE ou un agent de lyse.

**11.** Procédé de préparation d'un médicament selon les revendications 7 à 10, caractérisé en ce qu'un composé selon les revendications 1 à 5 ou son sel d'addition physiologiquement acceptable selon la revendication 6 est incorporé par voie non chimique, éventuellement en combinaison avec un inhibiteur de PDE ou un agent de lyse, dans un ou plusieurs supports et/ou diluants inertes courants.

**12.** Procédé de préparation des composés selon les revendications 1 à 6, caractérisé en ce que

a) un composé de formule

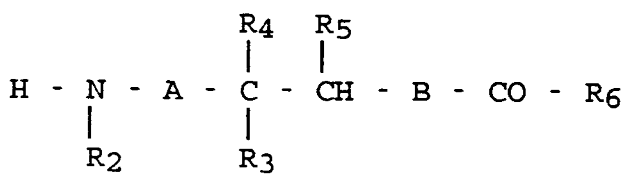

(II)

dans laquelle

$R_2$ à $R_6$, A et B sont définis comme dans l'une des revendications 1 à 5, est acylé avec un dérivé d'acide sulfonique de formule

$R_1$ - $SO_2X$ (III)

dans laquelle

$R_1$ est défini comme dans l'une des revendications 1 à 5 et

X représente un groupe partant nucléophile tel qu'un atome d'halogène ou un groupe alcoxy, par exemple un atome de chlore ou de brome, un groupe méthoxy ou éthoxy, ou

b) pour la préparation de composés de formule I dans laquelle $R_6$ représente un groupe hydroxyle, un reste protecteur est clivé d'un composé de formule

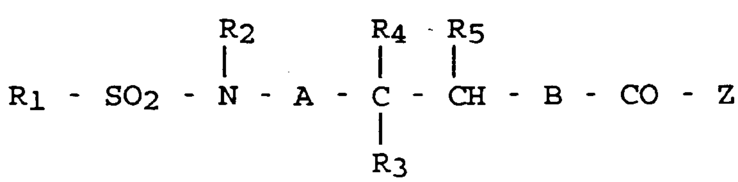

(IV)

dans laquelle

$R_1$ à $R_5$, A et B sont définis comme dans l'une des revendications 1 à 5 et
Z représente un groupe protecteur clivable par hydrolyse, thermolyse ou hydrogénolyse pour un groupe carboxyle ou un dérivé fonctionnel du groupe carboxyle, ou
c) pour la préparation de composés de formule I dans laquelle $R_4$ et $R_5$ représentent chacun un atome d'hydrogène, un composé de formule

$$R_1 - SO_2 - N(R_2) - A - C(R_3) = CH - B - CO - R_6 \quad (V)$$

dans laquelle
$R_1$ à $R_3$, $R_6$, A et B sont définis comme dans l'une des revendications 1 à 5, est hydrogéné ou
d) pour la préparation de composés de formule I dans laquelle $R_4$ et $R_5$ représentent ensemble une liaison carbone-carbone, un composé de formule

$$R_1 - SO_2 - N(R_2) - A - CO - R_3 \quad (VI)$$

dans laquelle
$R_1$ à $R_3$ et A sont définis comme dans l'une des revendications 1 à 5 est mis à réagir avec un composé de formule

$$W - CH(R_5') - B - CO - R_6 \quad (VII)$$

dans laquelle
B et $R_6$ sont définis comme dans l'une des revendications 1 à 5,
$R_5'$ représente un atome d'hydrogène ou un groupe alkyle de 1 à 3 atomes de carbone et
W représente un reste halogénure de triphénylphosphonium, un reste acide dialkylphosphonique ou un reste halogénure de magnésium, puis est éventuellement déshydraté et si on le souhaite un composé ainsi obtenu de formule I dans laquelle
$R_2$ représente un atome d'hydrogène est ensuite converti par alkylation en un composé correspondant de formule I dans laquelle $R_2$ représente un groupe alkyle, ou
un composé ainsi obtenu de formule I dans laquelle $R_6$ représente un groupe hydroxyle est converti par estérification ou amidification en un composé correspondant de formule I dans laquelle $R_6$ représente un groupe alcoxy, amino, alkylamino ou dialkylamino ou
un composé ainsi obtenu de formule I dans laquelle $R_4$ et $R_5$ représentent ensemble une liaison carbone-carbone est résolu en ses isomères cis et trans ou
un composé de formule I ainsi obtenu est résolu en ses énantiomères ou
un composé de formule I ainsi obtenu est converti en ses sels d'addition, en particulier en ses sels d'addition physiologiquement acceptables avec des bases inorganiques ou organiques.